# EUROPEAN PATENT SPECIFICATION

(11) **EP 3 423 070 B1**
(45) Date of publication and mention of the grant of the patent: **04.05.2022**
(21) Application number: 17760888.2
(22) Date of filing: 03.03.2017
(51) Int. Cl.: A61K 35/12, A61K 35/50, A61P 37/02, A61P 37/06

(54) **3-D COLLAGEN SCAFFOLD-GENERATED EXOSOMES AND USES THEREOF**
3D-KOLLAGENGERÜSTERZEUGTE EXOSOMEN UND VERWENDUNGEN DAVON
EXOSOMES GÉNÉRÉS PAR ÉCHAFAUDAGE DE COLLAGÈNE 3D ET LEURS UTILISATIONS

(30) Priority: 03.03.2016 US 201662302952 P
(43) Date of publication of application: 09.01.2019
(73) Proprietor: Henry Ford Health System, Detroit, MI 48202 (US)
(72) Inventor: ZHANG, Zhenggang, Troy Michigan 48085 (US); XIONG, Ye, Troy Michigan 48098 (US); CHOPP, Michael, Southfield Michigan 48034 (US)
(74) Representative: Cohausz & Florack
(86) International application number: PCT/US2017/020636
(87) International publication number: WO 2017/152035

(56) References cited:
- EP-A2- 2 745 840
- WO-A1-2015/179227
- US-A1- 2004 023 370
- US-A1- 2012 156 230
- US-A1- 2014 186 316
- US-A1- 2015 190 429
- US-A1- 2015 216 899
- YANLU ZHANG ET AL: "CONCLUSIONS", JOURNAL OF NEUROSURGERY, vol. 122, no. 4, 16 January 2015 (2015-01-16), pages 856-867, XP055567084, US ISSN: 0022-3085, DOI: 10.3171/2014.11.JNS14770
- DONG-KI KIM ET AL: "Chromatographically isolated CD63 + CD81 + extracellular vesicles from mesenchymal stromal cells rescue cognitive impairments after TBI", PNAS, vol. 113, no. 1, 5 January 2016 (2016-01-05), pages 170-175, XP055324139, US ISSN: 0027-8424, DOI: 10.1073/pnas.1522297113
- YE XIONG ET AL: "Investigational agents for treatment of traumatic brain injury", EXPERT OPINION ON INVESTIGATIONAL DRUGS, vol. 24, no. 6, 1 March 2015 (2015-03-01), pages 743-760, XP055605889, UK ISSN: 1354-3784, DOI: 10.1517/13543784.2015.1021919
- DUNYUE LU ET AL: "COLLAGEN SCAFFOLDS POPULATED WITH HUMAN MARROW STROMAL CELLS REDUCE LESION VOLUME AND IMPROVE FUNCTIONAL OUTCOME AFTER TRAUMATIC BRAIN INJURY", NEUROSURGERY., vol. 61, no. 3, 1 September 2007 (2007-09-01), pages 596-603, XP055605886, US ISSN: 0148-396X, DOI: 10.1227/01.NEU.0000290908.38438.B2

## Description

### TECHNICAL FIELD

The present disclosure relates to exosomes generated from a variety of stem cells and somatic cells, for example, mesenchymal stem cells (MSCs), for example, human MSCs, grown in 2D cell culture and/or 3D cell-scaffolds, for use in the prevention, treatment and cognitive recovery after traumatic brain injury (TBI). The present disclosure provides illustrative uses of the exemplified compositions comprising exosomes to promote robust neurovascular remodeling in the injured brain, reduce neuroinflammation in the injured brain, and therefore, the cell-free exosomes described herein may represent a novel treatment for TBI.

### BACKGROUND

Traumatic brain injury (TBI) is a major cause of death and long-term disability worldwide (Marklund and Hillered, 2011). The Demographics and Clinical Assessment Working

The Group of the International and Interagency Initiative toward Common Data Elements for Research on Traumatic Brain Injury and Psychological Health has formed an expert group that proposed the following definition of TBI: "TBI is defined as an alteration in brain function, or other evidence of brain pathology, caused by an external force." Menon, D. K. et al., "Position Statement: Definition of Traumatic Brain Injury" (2010) Arch. Phys. Med. Rehabil., Vol 91(11), pp 1637-1640. Within this definition the experts have stated that an alteration in brain function can be defined as one of the following clinical signs; (a) any period of loss of or a decreased LOC; (b) any loss of memory for events immediately before (retrograde amnesia) or after the injury (PTA); (c) neurologic deficits (weakness, loss of balance, change in vision, dyspraxia paresis/plegia [paralysis], sensory loss, aphasia, etc.); and (d) any alteration in mental state at the time of the injury (confusion, disorientation, slowed thinking, etc.). In addition, according to the definition of TBI, "other evidence of brain pathology" can include such evidence that may include visual, neuroradiologic, or laboratory confirmation of damage to the brain. Finally, the definition of "caused by an external force" may include any of the following events: (a) the head being struck by an object; (b) the head striking an object; (c) the brain undergoing an acceleration/deceleration movement without direct external trauma to the head; (d) a foreign body penetrating the brain; (e) forces generated from events such as a blast or explosion and (f) or other force yet to be defined.

Some of the symptoms associated with TBI, may include, but not limited to: Difficulty in initiating, organizing and completing tasks; Inconsistency in recall of information; Difficulty in using appropriate judgment; Difficulty with long-term memory; Difficulty with short-term memory; Difficulty in maintaining attention and concentration; Difficulty with flexibility in thinking, reasoning and problem-solving; Difficulty with orientation to person, places and/or time; Difficulty with speed of processing information; Exhibits gaps in task analysis; Difficulty in initiating, maintaining, restructuring and terminating conversation; Difficulty in maintaining the topic of conversation; Difficulty in discriminating relevant from irrelevant information; Difficulty in producing relevant speech; Difficulty responding to verbal communication in a timely, accurate, and efficient manner; Difficulty in understanding verbal information; Difficulty with word retrieval; Difficulty with articulation (which may include apraxia and/or dysarthria); 9. Difficulty with voice production (such as intensity, pitch and/or quality); Difficulty in producing fluent speech; Difficulty in formulating and sequencing ideas; Difficulty with abstract and figurative language; Difficulty with perseverated speech (repetition of words, phrases, and topics); Difficulty using appropriate syntax; Difficulty using language appropriately (such as requesting information, predicting, debating, and using humor); Difficulty in understanding and producing written communication; Difficulty with noise overload; Difficulty in interpreting subtle verbal and nonverbal cues during conversation; Difficulty in perceiving, evaluating and using social cues and context appropriately; Difficulty in initiating and sustaining appropriate peer and family relationships; Difficulty in demonstrating age-appropriate behavior; Difficulty in coping with over-stimulating environments; Denial of deficits affecting performance; Difficulty in establishing and maintaining self-esteem; Difficulty with using self-control (verbal and physical aggression); Difficulty with speaking and acting impulsively; Difficulty in initiating activities; Difficulty in adjusting to change; Difficulty in compliance with requests; Difficulty with hyperactivity; Intensification of pre-existent maladaptive behaviors and/or disabilities; Exhibits short-term or long-term physical disabilities; Displays seizure activity; Difficulty in spatial orientation (visual motor/ perceptual); Difficulty with mobility and independence (to include problems in balance, strength, muscle tone, equilibrium and gross motor skills); Difficulty with vision (which may include tracking, blind spots and/or double vision); Difficulty with dizziness (vertigo); Difficulty with auditory skills (which may include hearing loss and/or processing problems); Difficulty with fine motor skills (dexterity); Difficulty in speed of processing and motor response time; Difficulty with skills that affect eating and speaking (voluntary and involuntary); Difficulty with bowel and/or bladder control; Displays premature puberty; Loss of stamina and/or sense of fatigue; and Difficulty in administering self-care (such as independent feeding, grooming and toileting).

Effective pharmacologic treatments have not been identified from clinical trials in TBI (Narayan et al., 2002; Skolnick et al., 2014; Wright et al., 2014; Xiong et al., 2013). There is a compelling need to develop therapeutic approaches designed to improve functional recovery after TBI. Multipotent mesenchymal stem cells (MSCs) are a heterogeneous subpopulation consisting of mesenchymal stem and progenitor cells that can be harvested from bone marrow, umbilical cord blood, peripheral blood, adipose tissue and skin, as well as other organs (Ho et al., 2008; Walker et al., 2009). Administration of MSCs have shown promise as an effective therapy for brain injuries in experimental models of TBI and stroke (Chen et al., 2001a; Chen et al., 2003; Chopp and Li, 2002; Li et al., 2002; Li and Chopp, 2009; Lu et al., 2001c; Mahmood et al., 2004a, f; Nichols et al., 2013) and potentially in clinical settings (Cox et al., 2011; Doeppner and Hermann, 2014; Zhang et al., 2008). MSC-seeded collagen scaffolds that provide a three-dimensional (3D) environment to mimic the natural extracellular matrix have demonstrated therapeutic potential in preclinical studies of TBI (Lu et al., 2007; Mahmood et al., 2011; Mahmood et al., 2014a; Mahmood et al., 2014d; Qu et al., 2011; Qu et al., 2009; Xiong et al., 2009). Despite this therapeutic potential previous studies show that only a small proportion of transplanted MSCs actually survive and few MSCs differentiate into neural cells in injured brain tissues (Li et al., 2001; Lu et al., 2001c). The predominant mechanisms by which MSCs participate in brain remodeling and functional recovery are likely related to their secretion-based paracrine effect rather than a cell replacement effect (Chopp and Li, 2002; Li and Chopp, 2009). Our previous in vitro data show that collagen scaffolds stimulate human MSCs (hMSCs) to express multiple factors related to angiogenesis and neurogenesis, and signal transduction, which may contribute to hMSC survival, tissue repair, and functional recovery after TBI (Qu et al., 2011). Treatment of TBI with 3D collagen scaffolds impregnated with hMSCs significantly decreases functional deficits, promotes neurovascular remodeling, suppresses expression of axonal growth inhibitory molecules (for example, neurocan and Nogo-A) compared to the hMSCs group (Mahmood et al., 2011; Mahmood et al., 2014a; Mahmood et al., 2014d; Qu et al., 2011; Xiong et al., 2009).

Exosomes are endosomal origin small-membrane vesicles with a size of approximately 30 to 120 nm in diameter (Simons and Raposo, 2009; Vlassov et al., 2012). They are released by almost all cell types and contain not only proteins and lipids, but also messenger RNAs and micro RNAs (miRNAs) (Barteneva et al., 2013). Increasing evidence indicates that exosomes have a pivotal role in cell-to-cell communication (Pant et al., 2012). In contrast to transplanted exogenous MSCs, nanosized exosomes derived from MSCs do not proliferate, are less immunogenic and easier to store and deliver than MSCs (Lai et al., 2011). Recent studies indicate that exosomes and microvesicles derived from multipotent MSCs have therapeutic promise in cardiovascular, kidney, liver and lung diseases (Akyurekli et al., 2015; Borges et al., 2013; Cosme et al., 2013; Lai et al., 2011; Lai et al., 2015; Liang et al., 2014; Masyuk et al., 2013; Yu et al., 2014). Exosomes generated from rat MSCs improve functional recovery in rats after stroke (Xin et al., 2013b) and TBI (Zhang et al., 2015). However, whether exosomes generated from hMSCs promote neurovascular remodeling and functional recovery after TBI and whether exosomes derived from hMSCs cultured under 2-dimensional (2D) or (3D) conditions have any differential therapeutic effect, have not been investigated. In the present disclosure, we investigated the effects of exosomes generated from hMSCs cultured in 2D conventional condition or in 3D collagen scaffolds on cognitive and sensorimotor functional recovery as well as the potential mechanisms underlying therapeutic effects in rats after TBI.

Many molecules that have been individually tested effective in preclinical TBI models have not shown efficacy in clinical trials (McConeghy et al., 2012), suggesting that novel approaches may be required to target complex multiple secondary injury mechanisms involved in the TBI pathophysiology (Loane et al., 2015; Paterniti et al., 2015; Vink and Nimmo, 2009).

Zhang et al., J Neurosurg, vol. 122, 2015, pages 856-867 describes the effect of exosomes derived from multipluripotent mesenchymal stromal cells on functional recovery and neurovascular plasticity in rats after traumatic brain injury.

Kim et al., PNAS, vol. 113, 2016, pages 170-175 describes chromatographically isolated CD63⁺CD81⁺ extracellular vesicles from mesenchymal stromal cells that rescue cognitive impairments after TBI.

Xiong et al., Expert Opin Investig Drugs, vol. 24, pages 743-760 describes investigational agents for treatment of traumatic brain injury.

Lu et al., Neurosurgery, vol. 61, 2007, pages 596-603 describes collagen scaffolds populated with human marrow stromal cells that reduce lesion volume and improve functional outcome after traumatic brain injury.

WO 2015/179227 A1 describes compositions comprising exosome subpopulations and methods of their use in subjects having certain disorders including lung disorders, cardiovascular disorders, renal disorders and ischemic neural disorders.

### SUMMARY

The invention is defined by the appended claims.

The present invention provides a composition comprising exosomes obtained from human mesenchymal stem cells grown in three-dimensional cell culture comprising three-dimensional collagen scaffolds for use in treating a subject suffering from a traumatic brain injury (TBI).

In some embodiments, the composition comprises from about 1 × 10⁹ to about 1 × 10¹⁵ exosomes per kg body weight of the subject. In some embodiments, the composition is administered to the subject intravenously, subcutaneously, cutaneously, intraperitoneally, intraarterially, intracerebrally, intrathecally, intracerebroventricularly, or intranasally. In some embodiments, the composition is administered within 24 hours of developing the TBI. In some embodiments, treating a subject suffering from a traumatic brain injury comprises treating or preventing one or more symptoms of TBI. In some embodiments, one or more symptoms of TBI include one or more of:
(i) any period of loss of or a decreased level of consciousness;
(ii) loss of memory for events immediately before or after the injury;
(iii) a neurologic deficit, selected from the group consisting of: weakness, loss of balance, change in vision, dyspraxia, paresis, plegia, sensory loss, and aphasia;
(iv) an alteration in mental state at the time of the injury selected from confusion, disorientation, and slowed thinking;
(v) visual, neuroradiologic, or laboratory confirmation of damage to the brain;
(vi) Difficulty with long-term or short-term memory;
(vii) Difficulty in maintaining attention and concentration;
(viii) Difficulty with flexibility in thinking, reasoning and problem-solving;
(ix) Difficulty with orientation to person, places and/or time;
(x) Difficulty with speed of processing information;
(xi) Difficulty in initiating, maintaining, restructuring and terminating conversation; Difficulty in maintaining the topic of conversation; Difficulty in discriminating relevant from irrelevant information; Difficulty in producing relevant speech; Difficulty responding to verbal communication in a timely, accurate, and efficient manner; Difficulty in understanding verbal information;
(xii) Difficulty with word retrieval; Difficulty with articulation; Difficulty with voice production selected from the group consisting of intensity, pitch and/or quality of voice; Difficulty in producing fluent speech; Difficulty in formulating and sequencing ideas; Difficulty with abstract and figurative language; Difficulty with perseverated speech; Difficulty using appropriate syntax;
(xiii) Difficulty in understanding and producing written communication;
(xiv) Difficulty with noise overload;
(xv) Difficulty in interpreting subtle verbal and nonverbal cues during conversation;
(xvi) Difficulty in initiating and sustaining appropriate peer and family relationships;
(xvii) Difficulty in coping with over-stimulating environments;
(xviii) Difficulty with using self-control selected from verbal and physical aggression;
(xix) Difficulty with speaking and acting impulsively;
(xx) Difficulty in initiating activities; Difficulty in adjusting to change; Difficulty in compliance with requests;
(xxi) Hyperactivity; Intensification of pre-existent maladaptive behaviors and/or disabilities; Short-term or long-term physical disabilities; seizure activity; Difficulty in spatial orientation; Difficulty with mobility and independence; Problems in balance, strength, muscle tone, equilibrium and gross motor skills; Vertigo; Hearing loss and/or auditory processing problems; Difficulty with fine motor skills; Difficulty in speed of processing and motor response time; Difficulty with bowel and/or bladder control; premature puberty; Loss of stamina and/or sense of fatigue; and Difficulty in administering self-care.

In some embodiments, treating a subject suffering from a TBI comprises at least one or more of: (1) promotion of sensorimotor functional recovery; (2) enhancement of spatial learning; (3) an increase in vascular density and angiogenesis; (4) increased neurogenesis in the DG; and (5) significant reduction in brain inflammation.

In some instances, exosomes derived from any autologous, or allogeneic human exosome producing cell, for example, stem cells (embryonic stem cells, induced pluripotent stem cells, umbilical cord stem cells, neural stem cells, hematopoietic stem cells, hair follicle stem cells, and other somatic stem cells) and somatic cells, (for example, fibroblasts, Schwann cells, microglia, lymphocytes, dendritic cells, mast cells, and endothelial cells) grown in two-dimension and/or three-dimension cell culture can be used to treat or prevent traumatic brain injury, or symptoms associated with TBI. In some preferred instances, exosomes derived from multipotent human mesenchymal stem cells or multipotent human mesenchymal stromal cells (used interchangeably herein) (hMSCs) grown in two-dimensional and/or three-dimensional cell culture improve functional outcome after experimental traumatic brain injury (TBI). The present disclosure provides novel and unexpected findings related to the question whether systemic administration of cell-free exosomes generated from MSCs cultured in 2-dimensional (2D) conventional conditions or in 3-dimensional (3D) cell culture conditions, for example, 3D collagen scaffolds, promote functional recovery and neurovascular remodeling in mammals after TBI. In a first aspect, the present disclosure provides a method for treating, and/or ameliorating one or more symptoms of TBI, the method comprising administering a safe and therapeutically effective amount of a composition comprising exosomes obtained from an exosome producing cell grown in two-dimensional cell culture or three-dimensional cell culture to the subject in need thereof. In a related aspect, the present disclosure provides a method for treating, and/or ameliorating one or more symptoms of TBI, the method comprising administering a safe and therapeutically effective amount of a composition comprising exosomes obtained from mesenchymal stem cells grown in three-dimensional cell culture, for example, 3D collagen scaffolds, to a subject in need of treatment and/or amelioration of one or more TBI symptoms. In exemplary embodiments, the mesenchymal stem cells are human mesenchymal stem cells.

### BRIEF DESCRIPTION OF THE DRAWINGS

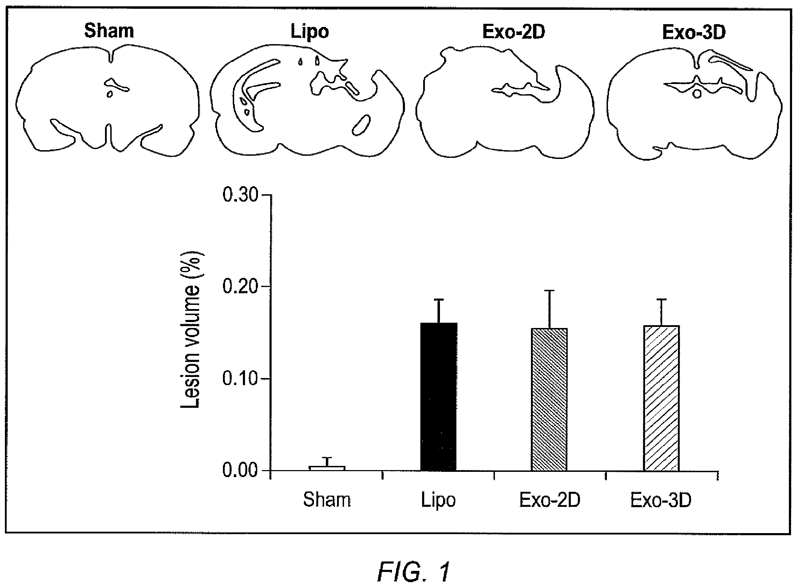

FIG. 1 shows the effect of treatment with exosomes derived from human mesenchymal stromal cells (hMSCs) on cortical lesion volume examined 35 days after traumatic brain injury (TBI). TBI caused significant cortical tissue loss in the liposome-treated rats. The bar graph showing that exosome treatments did not reduce lesion volume compared to the liposome-treated groups (p > 0.05). Scale bar = 3 mm. Data represent mean ± SD. N = 8/group.

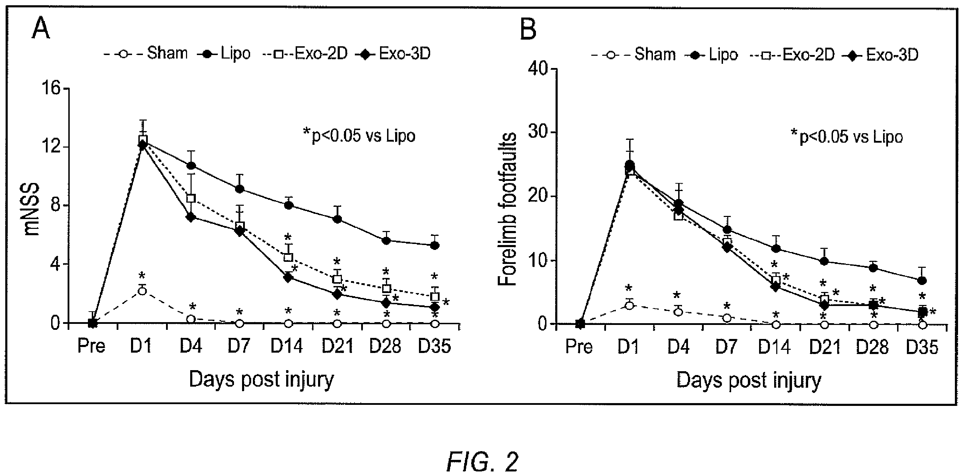

FIG. 2 shows the treatment with exosomes derived from hMSCs significantly improves sensorimotor functional recovery measured by mNSS (A), and right forelimb foot fault test (B) in rats after TBI. ^{∗}p<0.05 vs liposome group. Data represent mean ± SD. N=8/group.

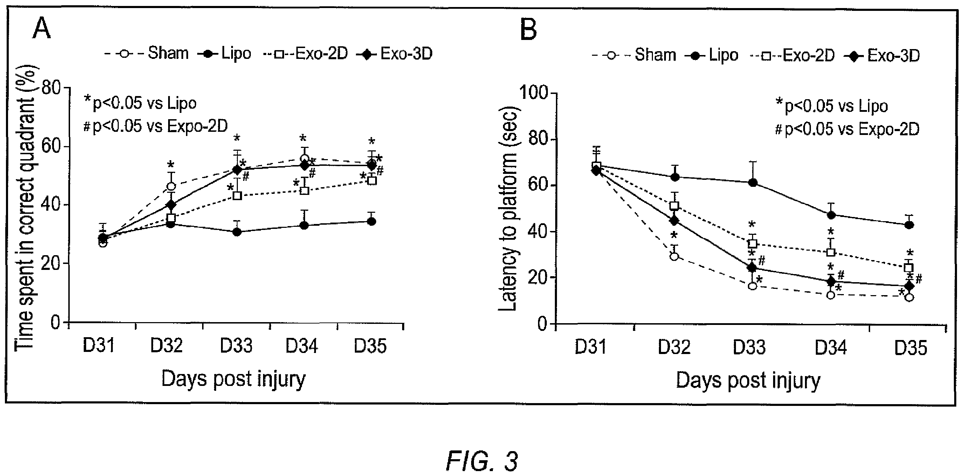

FIG. 3 shows the treatment with exosomes derived from hMSCs significantly improves spatial learning in the Morris water maze text measured by percentage time spent (A), and latency to find the hidden platform (B) in the correct quadrant by rats after TBI. ^{∗}p<0.05 vs Lipo group. #p<0.05 vs Exo-2D group. Data represent mean ± SD. N = 8/group.

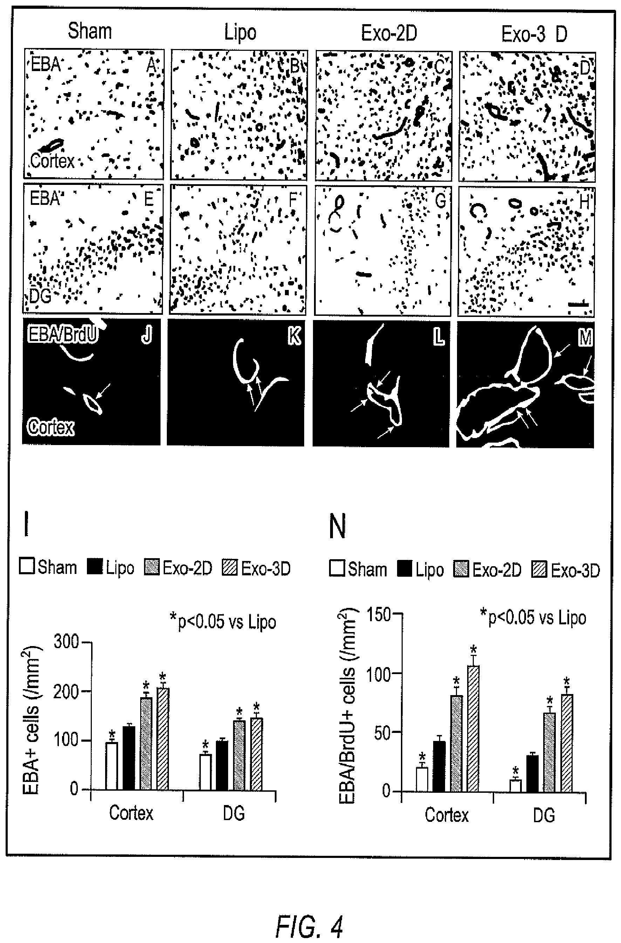

FIG. 4 shows the treatment with exosomes derived from hMSCs significantly increases brain vascular density and angiogenesis in rats after TBI. EBA staining was performed for detection of mature vasculature at day 35 after TBI in the lesion boundary zone and dentate gyrus (DG) from rat brains in the sham group (A and E), liposome-treated group (B and F), and exosome-treated groups (C and G for exosomes derived from hMSCs grown in 2-dimensional cell culture (Exo-2D); D and H for exosomes derived from hMSCs grown in 3-dimensional cell culture (Exo-3D)). Double staining for EBA (green) and BrdU (red) to identify newly formed mature vessels (arrows, J-M) in the brain at day 35 after TBI. Scale bar (F, J) =25 µm. Data in bar graph (I, N) represent mean ± SD. ^{∗}p < 0.05 vs liposome group. N = 8/group.

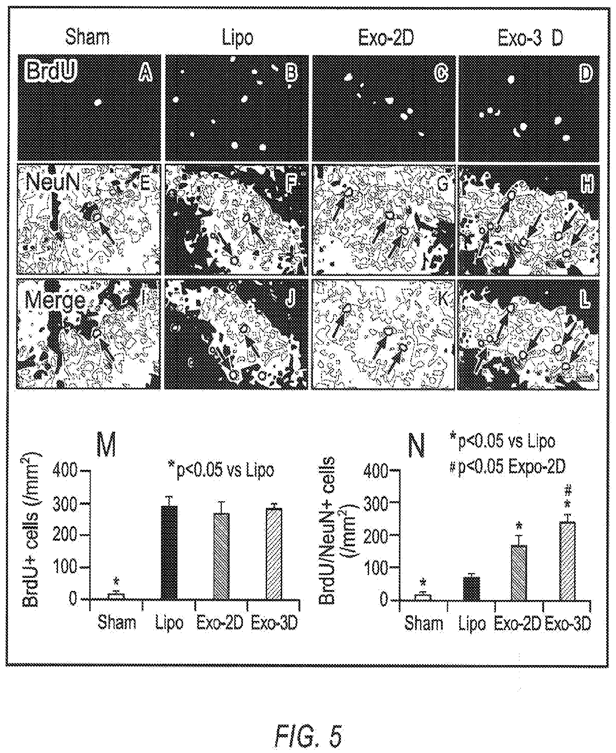

FIG. 5 shows the treatment with exosomes derived from hMSCs significantly increases neurogenesis in the DG of rats sacrificed at day 35 after TBI. Double staining with BrdU (red, **A-D**)/NeuN (green, **E-H**) was performed to identify newborn mature neurons indicated by yellow arrows (**I-L**, arrows). Scale bar = 25 µm (**L**). Data in bar graphs (**M, N**) represent mean ± SD. ^{∗}p < 0.05 vs liposome group. #p < 0.05 vs Exo-2D group. N = 8/group.

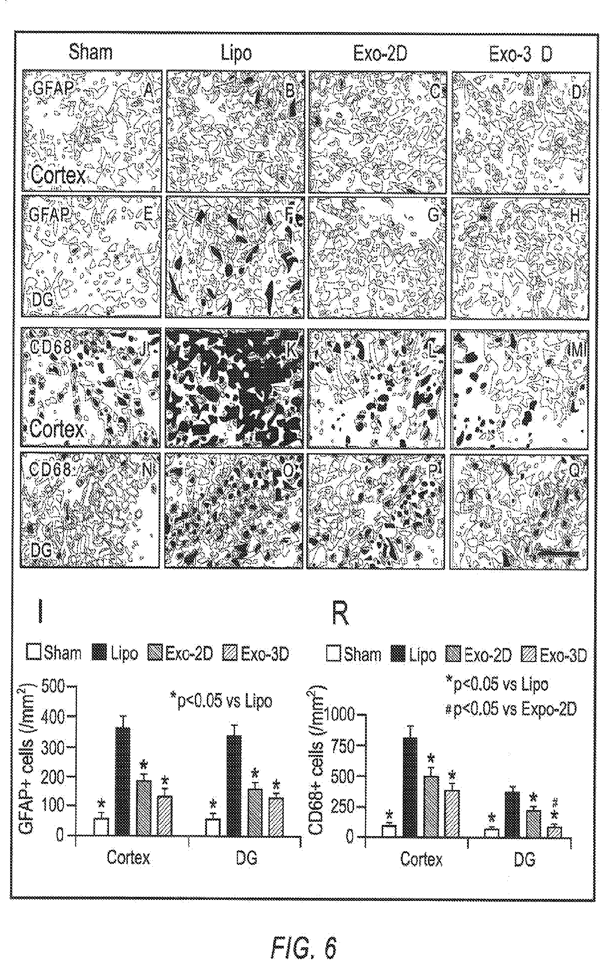

FIG. 6 shows the treatment with exosomes derived from hMSCs significantly reduces the number of activated GFAP+ astrocytes and CD68+ microglia/macrophages in the brain of rats sacrificed at day 35 after TBI. GFAP staining for reactive astrocytes (**A-H**). CD68 staining for activated microglia/macrophages (**J-Q**). Scale bar = 50 µm (**Q**). Data in bar graphs (**I, R**) represent mean ± SD. N = 8/group.

### DETAILED DESCRIPTION

The invention is defined by the appended claims.

The present invention provides a composition comprising exosomes obtained from human mesenchymal stem cells grown in three-dimensional cell culture comprising three-dimensional collagen scaffolds for use in treating a subject suffering from a traumatic brain injury (TBI).

In some embodiments, the composition comprises from about 1 × 10⁹ to about 1 × 10¹⁵ exosomes per kg body weight of the subject. In some embodiments, the composition is administered to the subject intravenously, subcutaneously, cutaneously, intraperitoneally, intraarterially, intracerebrally, intrathecally, intracerebroventricularly, or intranasally. In some embodiments, the composition is administered within 24 hours of developing the TBI. In some embodiments, treating a subject suffering from a traumatic brain injury comprises treating or preventing one or more symptoms of TBI. In some embodiments, one or more symptoms of TBI include one or more of:
(xxii) any period of loss of or a decreased level of consciousness;
(xxiii) loss of memory for events immediately before or after the injury;
(xxiv) a neurologic deficit, selected from the group consisting of: weakness, loss of balance, change in vision, dyspraxia, paresis, plegia, sensory loss, and aphasia;
(xxv) an alteration in mental state at the time of the injury selected from confusion, disorientation, and slowed thinking;
(xxvi) visual, neuroradiologic, or laboratory confirmation of damage to the brain;
(xxvii) Difficulty with long-term or short-term memory;
(xxviii) Difficulty in maintaining attention and concentration;
(xxix) Difficulty with flexibility in thinking, reasoning and problem-solving;
(xxx) Difficulty with orientation to person, places and/or time;
(xxxi) Difficulty with speed of processing information;
(xxxii) Difficulty in initiating, maintaining, restructuring and terminating conversation; Difficulty in maintaining the topic of conversation; Difficulty in discriminating relevant from irrelevant information; Difficulty in producing relevant speech; Difficulty responding to verbal communication in a timely, accurate, and efficient manner; Difficulty in understanding verbal information;
(xxxiii) Difficulty with word retrieval; Difficulty with articulation; Difficulty with voice production selected from the group consisting of intensity, pitch and/or quality of voice; Difficulty in producing fluent speech; Difficulty in formulating and sequencing ideas; Difficulty with abstract and figurative language; Difficulty with perseverated speech; Difficulty using appropriate syntax;
(xxxiv) Difficulty in understanding and producing written communication;
(xxxv) Difficulty with noise overload;
(xxxvi) Difficulty in interpreting subtle verbal and nonverbal cues during conversation; (xxxvii) Difficulty in initiating and sustaining appropriate peer and family relationships; (xxxviii)Difficulty in coping with over-stimulating environments;
(xxxix) Difficulty with using self-control selected from verbal and physical aggression;
(xl) Difficulty with speaking and acting impulsively;
(xli) Difficulty in initiating activities; Difficulty in adjusting to change; Difficulty in compliance with requests;
(xlii) Hyperactivity; Intensification of pre-existent maladaptive behaviors and/or disabilities; Short-term or long-term physical disabilities; seizure activity; Difficulty in spatial orientation; Difficulty with mobility and independence; Problems in balance, strength, muscle tone, equilibrium and gross motor skills; Vertigo; Hearing loss and/or auditory processing problems; Difficulty with fine motor skills; Difficulty in speed of processing and motor response time; Difficulty with bowel and/or bladder control; premature puberty; Loss of stamina and/or sense of fatigue; and Difficulty in administering self-care.

In some embodiments, treating a subject suffering from a TBI comprises at least one or more of: (1) promotion of sensorimotor functional recovery; (2) enhancement of spatial learning; (3) an increase in vascular density and angiogenesis; (4) increased neurogenesis in the DG; and (5) significant reduction in brain inflammation.

Without limitation, in some embodiments the present disclosure provides a composition for use in treatments which reduce neurological deficits after traumatic brain injury and promote significant spontaneous sensorimotor functional recovery occurring after TBI. Thus, in some instances the present disclosure provides a method for treating and/or ameliorating at least one symptom associated with traumatic brain injury in a subject, the method comprising administering a safe and therapeutically effective amount of a composition comprising exosomes generated from an exosome producing cell which has been grown in two-dimensional (2D) and/or three-dimensional (3D) cultures. As used herein, the term "an exosome producing cell" can include any mammalian stem cell or somatic cell that is capable of producing exosomes. In some instances, exosome producing cells can include, without limitation, stem cells (embryonic stem cells, induced pluripotent stem cells, hematopoietic stem cells, hair follicle stem cells, and other somatic stem cells) and somatic cells. In some instances, the exosome producing cell, is a mammalian exosome producing cell, preferably a human exosome producing cell. According to the claimed invention, the exosome producing cell is a mesenchymal stem or stromal cell. The terms "mesenchymal stem cells" and "mesenchymal stromal cells" are used interchangeably herein, and are both termed "MSCs". In various instances the term "MSC" or "MSCs" refer to mesenchymal stem cells and mesenchymal stromal cells derived from mammalian species. According to the claimed invention, the term "MSC" or "MSCs" refer to mesenchymal stem cells and mesenchymal stromal cells derived from humans. In some embodiments, human MSCs are derived from bone marrow. MSCs of the present invention have self-renewal potential. These cells express markers for mesenchymal or endothelial cells (CD105, CD73, and CD90) as well as adhesion molecules (CD106, CD166 and CD29) (Javazon et al., 2004; Dominici et al., 2006), but do not express hematopoietic stem cell markers (CD11, CD14, CD34, CD45, CD79, CD19 and HLA-DR). MSCs can differentiate not only into mesodermal cells but also into endodermal and ectodermal cells (Sanchez-Ramos et al., 2000; Phinney and Prockop, 2007; Uccelli et al., 2008).

In various instances, the methods of the present disclosure that the composition of the invention is for use in provide administration of a composition comprising exosomes generated from an exosome producing cell grown in 2D or 3D cultures to the subject in need thereof. Administration can be performed using any suitable method that preserves the functionality and therapeutic benefit of the exosomes, for example, when administered to a human subject, the composition may be administered parenterally. There is a great effort underway to develop therapies which increase vascular density and angiogenesis, functionally resulting in brain remodeling and enhance recovery of neurological function after an injury/disease. In some embodiments, the administration of human mesenchymal stem cell exosomes to individuals who have suffered a traumatic brain injury, generally provides one or more of the following recuperative effects: (1) significant promotion of sensorimotor functional recovery; (2) enhancement of spatial learning; (3) an increase in vascular density and angiogenesis; (4) increased neurogenesis in the DG; and (5) significant reduction in brain inflammation.

In accordance with some embodiments, the present disclosure provides compositions for use in a method for the treatment and/or amelioration of a sign or symptom associated with the diseases and/or disorders described herein, and/or recuperation and cognitive recovery of a subject having experienced a TBI, the method comprising administering a composition comprising cell-free exosomes generated by exosome producing cells cultured under 2D and/or 3D conditions, preferably for example, a stem cell, or a somatic cell, for example human MSCs cultured under 2D and/or 3D conditions. In various embodiments, the administration to the subject with a TBI, of the compositions described herein can be systematic administration, for example, parenterally, for example, via intravenous, subcutaneous, cutaneous injection and/or infusion, intraarterial injection, intrathecal injection/infusion, and intracerebral injection and/or intracerebroventricular infusion or intranasally. In some instances, the administration of the cell-free exosomes generated by an exosome producing cell, for example, MSCs, for example human MSCs cultured under 2D and/or 3D conditions is initiated within 24 hours post injury of a TBI. In various embodiments, if administered within 24 hours from receiving the TBI, or experiencing one or more symptoms of TBI, the subject may have an unaltered cortical lesion volume compared to no treatment or treatment with another drug or medicament. The exact amount of exosomes required will vary from subject to subject, depending on the species, age, and general condition of the subject, the severity of the infection, the particular agent, its mode of administration, and the like. The compositions are preferably formulated in dosage unit form for ease of administration and uniformity of dosage. The expression "dosage unit form" as used herein refers to a physically discrete unit of agent appropriate for the patient to be treated. It will be understood, however, that the total daily usage of the exosomes and compositions will be decided by the attending physician within the scope of sound medical judgment. The specific effective dose level for any particular patient or organism will depend upon a variety of factors including the disorder being treated and the severity of the disorder; the activity of the specific composition employed; the specific composition employed; the age, body weight, general health, sex and diet of the patient; the time of administration, route of administration, and rate of excretion of the specific compound employed; the duration of the treatment; drugs used in combination or coincidental with the specific compound employed, and like factors well known in the medical arts. The term "patient" or "subject" used synonymously herein, means an animal, preferably a mammal, and most preferably a human

In various instances, administration of a composition comprising cell-free exosomes generated and isolated from exosome producing cells, for example mammalian stem cells, for example, MSCs, for example human MSCs, cultured under 2D and/or 3D conditions, to a subject in need thereof (i.e. having a TBI) may experience significant: 1) improvement in cognitive and sensorimotor functional recovery; 2) increase in the number of newborn mature neurons in the DG; 3) increase in the number of newborn endothelial cells in the lesion boundary zone and DG; 4) reduced neuroinflammation; and 5) exosomes generated from MSCs cultured in 3D condition provide better outcome in spatial learning compared to exosomes from 2D culture. Exosome treatments initiated 24 h post injury did not reduce lesion volume, suggesting that beneficial effects of exosomes is not attributed to direct neuroprotection, but, rather to neurovascular remodeling. Improved functional recovery after treatment of TBI with exosomes generated from and exosome producing cell, for example, MSCs is significantly associated with increased brain angiogenesis and neurogenesis as well as with reduced neuroinflammation. Based on results provided herein suggest that parenteral administration of exosomes generated from an exosome producing cell, for example, a stem cell, e.g. MSCs, may represent a novel cell-free therapeutic approach for treatment of TBI. In some instances, without limitation, administration of an effective amount of a composition comprising cell-free exosomes generated by an exosome producing cell, for example, a stem cell, e.g. MSCs for example, human MSCs cultured under 2D and/or 3D conditions can be administered to patients before or after the onset of TBI to reduce the neurological deficits associated with neurological disease, e.g. TBI and vascular complications caused by TBI.

The methods of the present disclosure that the compositions of the invention are for use in, are supported by experimental evidence of therapeutic benefit in Wistar rats that were subjected to TBI induced by controlled cortical impact; 24 hours later tail vein injection of exosomes derived from MSCs cultured under 2D or 3D conditions or an equal number of liposomes as a treatment control. The modified Morris water maze, neurological severity score and foot fault tests were employed to evaluate cognitive and sensorimotor functional recovery. Animals were sacrificed at 35 days after TBI. Histological and immunohistochemical analyses were performed for measurements of lesion volume, neurovascular remodeling (angiogenesis and neurogenesis), and neuroinflammation.

Compared with liposome-treated control, exosome-treatments did not reduce lesion size but significantly improved spatial learning at 33-35 days measured by the Morris water maze test, and sensorimotor functional recovery, i.e., reduced neurological deficits and foot fault frequency, observed at 14-35 days post injury (p < 0.05). Exosome treatments significantly increased the number of newborn endothelial cells in the lesion boundary zone and dentate gyrus, and significantly increased the number of newborn mature neurons in the dentate gyrus as well as reduced neuroinflammation. Exosomes derived from MSCs cultured in 3D scaffolds provided better outcome in spatial learning than exosomes from MSCs cultured in the 2D condition. In conclusion, MSC-generated exosomes significantly improve functional recovery in rats after TBI, at least in part, by promoting endogenous angiogenesis and neurogenesis and reducing neuroinflammation. Thus, exosomes derived from an exosome producing cell, for example a stem cell, e.g. MSCs, for example human MSCs, is a novel cell-free therapy for TBI, and an exosome producing cell, for example a stem cell, e.g. a MSC grown in a3D-scaffold, generated exosomes that can selectively enhance spatial learning.

As used herein, an "effective amount" is defined as the amount required to confer a therapeutic effect on the treated patient, and is typically determined based on age, surface area, weight, and condition of the patient. The interrelationship of dosages for animals and humans (based on milligrams per meter squared of body surface) is described by Freireich et al., Cancer Chemother. Rep., 50: 219 (1966). Body surface area may be approximately determined from height and weight of the patient. See, e.g., Scientific Tables, Geigy Pharmaceuticals, Ardsley, New York, 537 (1970). As used herein, "patient" refers to a mammal, including a human.

In various instances, exosomes (or their internal components thereof), isolated and obtained from an exosome producing cell, including without limitation, stem cells (embryonic stem cells, induced pluripotent stem cells, umbilical cord stem cells, neural stem cells, hematopoietic stem cells, e.g. MSCs, hair follicle stem cells, and other somatic stem cells) and somatic cells were grown or cultured in 2D cell culture or in 3D cell culture conditions, for example, in 3D biopolymer scaffolds, for example 3D collagen scaffolds, can be administered and dosed in accordance with good medical practice, taking into account the clinical condition of the individual patient, the site and method of administration, scheduling of administration, patient age, sex, body weight and other factors known to medical practitioners. The "pharmaceutically effective amount" for purposes herein is thus determined by such considerations as are known in the art, and may also include "therapeutically effective amounts" (also used synonymously) which is broadly used herein to mean an amount of the exosomes, that when administered to a patient, ameliorates, diminishes, improves or prevents a symptom of TBI. In some embodiments, the amount of the exosomes of the invention which constitutes a "therapeutically effective amount" will vary depending on the exosome density, the disease state and its severity, the age of the patient to be treated, and the like. Preferably, the exosomes are formulated in the composition in a therapeutically effective amount. A "therapeutically effective amount" refers to an amount effective, at dosages and for periods of time necessary, to achieve the desired therapeutic result to thereby influence the therapeutic course of a particular disease state. A therapeutically effective amount of an active agent may vary according to factors such as the disease state, age, sex, and weight of the individual, and the ability of the agent to elicit a desired response in the individual. Dosage regimens may be adjusted to provide the optimum therapeutic response. A therapeutically effective amount is also one in which any toxic or detrimental effects of the agent are outweighed by the therapeutically beneficial effects. In another embodiment, the active agent is formulated in the composition in a prophylactically effective amount. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

The amount or number of exosomes or their contents in the composition to provide a therapeutically effective amount may vary according to factors such as the disease state, age, sex, and weight of the individual. Dosage regimens may be adjusted to provide the optimum therapeutic response. For example, a single bolus of exosomes (or compositions containing the contents of said exosomes) may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

"Patient" or "Subject" are used interchangeably and for the purposes of the present invention includes humans and other animals, particularly mammals, and other organisms. Thus the methods are applicable to both human therapy and veterinary applications. More specifically, the patient is a mammal, and in some embodiments, the patient or subject is human.

"Treating" or "treatment" or "to treat" in the context of this specification means administration of an MSC derived exosome containing composition or formulation according to the invention to prevent, ameliorate or eliminate one or more symptoms associated with traumatic brain injury, as defined herein, or to treat one or more symptoms associated with TBI, for example: Difficulty in initiating, organizing and completing tasks; Inconsistency in recall of information; Difficulty in using appropriate judgment; Difficulty with long-term memory; Difficulty with short-term memory; Difficulty in maintaining attention and concentration; Difficulty with flexibility in thinking, reasoning and problem-solving; Difficulty with orientation to person, places and/or time; Difficulty with speed of processing information; Exhibits gaps in task analysis; Difficulty in initiating, maintaining, restructuring and terminating conversation; Difficulty in maintaining the topic of conversation; Difficulty in discriminating relevant from irrelevant information; Difficulty in producing relevant speech; Difficulty responding to verbal communication in a timely, accurate, and efficient manner; Difficulty in understanding verbal information; Difficulty with word retrieval; Difficulty with articulation (which may include apraxia and/or dysarthria); 9. Difficulty with voice production (such as intensity, pitch and/or quality); Difficulty in producing fluent speech; Difficulty in formulating and sequencing ideas; Difficulty with abstract and figurative language; Difficulty with perseverated speech (repetition of words, phrases, and topics); Difficulty using appropriate syntax; Difficulty using language appropriately (such as requesting information, predicting, debating, and using humor); Difficulty in understanding and producing written communication; Difficulty with noise overload; Difficulty in interpreting subtle verbal and nonverbal cues during conversation; Difficulty in perceiving, evaluating and using social cues and context appropriately; Difficulty in initiating and sustaining appropriate peer and family relationships; Difficulty in demonstrating age-appropriate behavior; Difficulty in coping with over-stimulating environments; Denial of deficits affecting performance; Difficulty in establishing and maintaining self-esteem; Difficulty with using self-control (verbal and physical aggression); Difficulty with speaking and acting impulsively; Difficulty in initiating activities; Difficulty in adjusting to change; Difficulty in compliance with requests; Difficulty with hyperactivity; Intensification of pre-existent maladaptive behaviors and/or disabilities; Exhibits short-term or long-term physical disabilities; Displays seizure activity; Difficulty in spatial orientation (visual motor/ perceptual); Difficulty with mobility and independence (to include problems in balance, strength, muscle tone, equilibrium and gross motor skills); Difficulty with vision (which may include tracking, blind spots and/or double vision); Difficulty with dizziness (vertigo); Difficulty with auditory skills (which may include hearing loss and/or processing problems); Difficulty with fine motor skills (dexterity); Difficulty in speed of processing and motor response time; Difficulty with skills that affect eating and speaking (voluntary and involuntary); Difficulty with bowel and/or bladder control; Displays premature puberty; Loss of stamina and/or sense of fatigue; and Difficulty in administering self-care (such as independent feeding, grooming and toileting). Furthermore, the terms "to treat" or "treatment" according to this invention include the treatment of symptoms of TBI, the prevention or the prophylaxis of TBI, the prevention or prophylaxis of one or more TBI symptoms, as well as the prevention or the prophylaxis of the consequences causing the symptoms.

"Prevent" or "preventing" or "prevention" refer to prevention or delay of the onset of the symptoms associated with TBI in a subject relative to the symptoms associated with TBI that would develop in the absence of the composition for use in treatment methods according to the invention. The prevention can be complete, e.g., the lack of functional or cognitive deficit after a TBI in a subject. The prevention can also be partial, such that the occurrence of one or more symptoms related to TBI in a subject that are fewer in number or severity than that which would have occurred without the present invention.

In a highly preferred embodiment of the composition for use according to the invention the prevention, amelioration and/or elimination one or more symptoms associated with traumatic brain injury symptoms can include: improvement in cognitive and sensorimotor functional recovery; 2) increase in the number of newborn mature neurons in the DG; 3) increase in the number of newborn endothelial cells in the lesion boundary zone and DG; 4) reduced neuroinflammation; and 5) better outcome in spatial learning.

Pharmaceutically acceptable refers to those properties and/or substances which are acceptable to the patient from a pharmacological/toxicological point of view and to the manufacturing pharmaceutical chemist from a physical/chemical point of view regarding composition, formulation, stability, patient acceptance, and bioavailability.

In various instances, therapeutically effective compositions or formulations containing exosomes obtained from cells grown in 2D or 3D cell culture conditions useful in the prevention and/or treatment of TBI, can be administered before, after or concurrently with the administration of another active agent used in the art to treat TBI symptoms, for example, oxygen, a diuretic, an anti-seizure drug, an anti-inflammatory drug, or a coma-inducing drug. "Concurrently" means sufficiently close in time to produce a combined effect (that is, concurrently can be simultaneously, or it can be two or more events occurring within a short time period before or after each other). In some embodiments, the administration of two or more compounds "concurrently" means that the two compounds are administered closely enough in time that the presence of one alters the biological effects of the other. The two compounds can be administered in the same or different formulations or sequentially. Concurrent administration can be carried out by mixing the compounds prior to administration, or by administering the compounds in two different formulations, for example, at the same point in time but at different anatomic sites or using different routes of administration.

In some embodiments, therapeutically effective compositions or formulations may contain whole exosomes, i.e. whole particles, partially disrupted exosomes, or exosome contents, i.e. the internal constituents of exosomes minus the exosome membrane. Accordingly, the disclosure further provides a pharmaceutical composition comprising exosomes (or their internal components thereof), isolated and obtained from an exosome producing cell, including without limitation, stem cells (embryonic stem cells, induced pluripotent stem cells, umbilical cord stem cells, neural stem cells, hematopoietic stem cells, e.g. MSCs, hair follicle stem cells, and other somatic stem cells) and somatic cells grown in 2D cell culture conditions and/or 3D cell culture conditions. In one illustrative embodiment, a pharmaceutical composition comprising exosomes isolated and obtained from MSCs grown in a 3D biopolymer, for example, a 3D-collagen scaffold, a 3D-agarose scaffold, a 3D-matrigel scaffold, a 3D-hydrogel, a 3D-microfiber scaffold, and mixtures thereof can be used for the prevention, amelioration and/or treatment of one or more symptoms associated with traumatic brain injury including: 1) improvement in cognitive and sensorimotor functional recovery; 2) increase in the number of newborn mature neurons in the DG; 3) increase in the number of newborn endothelial cells in the lesion boundary zone and DG; 4) reduced neuroinflammation; and 5) better outcome in spatial learning. In various embodiments, the 3D cell cultures used to grow the exosome producing cell of the present invention are supplemented with various growth factors and other nutrients, commonly used to grow such cells in cell culture.

The pharmaceutical composition may further comprise one or more pharmaceutically acceptable carriers, diluents, and/or excipients. The carrier(s), diluent(s) and/or excipient(s) must be acceptable in the sense of being compatible with the other ingredients of the formulation and not deleterious to the recipient thereof. In accordance with another aspect of the invention there is also provided a process for the preparation of a pharmaceutical formulation including admixing exosomes of the invention with one or more pharmaceutically acceptable carriers, diluents and/or excipients. In certain embodiments, the exosome containing compositions or formulations of the invention can contain further additives including, but not limited to, pH-adjusting additives, osmolarity adjusters, tonicity adjusters, anti-oxidants, reducing agents, and preservatives. Useful pH-adjusting agents include acids, such as hydrochloric acid, bases or buffers, such as sodium lactate, sodium acetate, sodium phosphate, sodium citrate, sodium borate, or sodium gluconate. Further, the compositions of the invention can contain microbial preservatives. Useful microbial preservatives include methylparaben, propylparaben, and benzyl alcohol. The microbial preservative is typically employed when the formulation is placed in a vial designed for multidose use. Other additives that are well known in the art include, e.g., detackifiers, anti-foaming agents, antioxidants (e.g., ascorbyl palmitate, butyl hydroxy anisole (BHA), butyl hydroxy toluene (BHT) and tocopherols, e.g., .alpha.-tocopherol (vitamin E)), preservatives, chelating agents (e.g., EDTA and/or EGTA), viscomodulators, tonicifiers (e.g., a sugar such as sucrose, lactose, and/or mannitol), flavorants, colorants, odorants, opacifiers, suspending agents, binders, fillers, plasticizers, lubricants, and mixtures thereof. The amounts of such additives can be readily determined by one skilled in the art, according to the particular properties desired.

In another embodiment of the disclosure, exosomes or pharmaceutically acceptable compositions containing exosomes described herein are used to treat or prevent TBI symptoms in a subject. As stated above, the exosomes are extracted from exosome producing cells, for example, MSCs that have been grown in 2D cell culture conditions or exosome producing cells, for example, MSCs that have been grown in 3D cell culture conditions, such as those exosome producing cells, for example, MSCs grown in 3D biopolymer scaffolds, for example, a 3D-collagen scaffold, a 3D-agarose scaffold, a 3D-matrigel scaffold, a 3D-hydrogel, a 3D-microfiber scaffold, and mixtures thereof. In various embodiments, a method for treating a subject suffering from a traumatic brain injury that the composition of the invention is for use in comprises administering a therapeutically effective amount of a composition comprising exosomes obtained from exosome producing cells, for example, MSCs grown in two-dimensional cell culture to the subject in need thereof. The present disclosure provides a composition for use in a method for treating a subject suffering from a traumatic brain injury. The method includes administering a therapeutically effective amount of a composition comprising exosomes obtained from exosome producing cells, for example, MSCs grown in a three-dimensional cell culture to the subject in need thereof. In each of these embodiments, the therapeutically effective amount of the composition may contain from about 1×10⁵ to about 1×10¹⁵ exosomes or from about 1×10⁹ to about 1×10¹² exosomes. In another embodiment of the invention, exosomes described herein are administered to the subject as needed to treat or prevent TBI symptoms in a human subject. The exosomes can be administered continuously or intermittently. In one embodiment, the exosome containing composition or formulation is administered to the subject more than once a day or once every 1, 2, 3, 4, 5, 6, or 7 days. In another embodiment, the exosome containing composition or formulation is administered to the subject no more than once a week, e.g., no more than once every two weeks, once a month, once every two months, once every three months, once every four months, once every five months, once every six months, or longer. In a further embodiment, the exosome containing composition or formulation is administered using two or more different schedules, e.g., more frequently initially (for example to build up to a certain level, e.g., once a day or more) and then less frequently (e.g., once a week or less).

The exosome containing composition or formulation can be administered 1 hour, 2 hours, 3 hours, 4 hours, 5 hours, 6 hours, 12 hours, 1 day, 2 days, 3 days, 4 days, 5 days, 6 days, 1 week, 2 weeks, 3 weeks, 4 weeks, or more prior to the administration of one or more secondary active agents (e.g., oxygen, a diuretic, an anti-seizure drug, or an anti-inflammatory drug), concurrently with, or administered 1 hour, 2 hours, 3 hours, 4 hours, 5 hours, 6 hours, 12 hours, 1 day, 2 days, 3 days, 4 days, 5 days, 6 days, 1 week, 2 weeks, 3 weeks, 4 weeks, or more after the administration of one or more secondary active agents (e.g., oxygen, a diuretic, an anti-seizure drug, an anti-inflammatory drug, or a coma-inducing drug). The secondary active agents (e.g., oxygen, a diuretic, an anti-seizure drug, or an anti-inflammatory drug) may be used in combination with the exosome containing composition or formulation of the present invention to prevent oxygen or nutrient loss in the brain, and to prevent bleeding and/or overly high internal pressures in the brain as a result of the TBI.

In some embodiments, the exosome containing composition or formulation of the present invention may be administered concurrently during surgery, or after surgery, or 1 hour, 2 hours, 3 hours, 4 hours, 5 hours, 6 hours, 12 hours, or 1 day prior to surgical intervention in a case of TBI, for example, surgery to relieve intracranial pressure or to remove blood clots or other neurological emergencies as a result of the TBI. In other embodiments, the exosome containing composition or formulation can be administered by any discontinuous administration regimen. In one example, the exosome containing composition or formulation may be administered once per day, once every two days, once every three days, every four days, every five days, every six days, every seven days, every eight days, every nine days, or every ten days, or longer after the onset of TBI symptoms, or in a suspected case of TBI, or prophylactically prior to an activity that may likely result in TBI. The administration can continue for one, two, three, or four weeks, or one, two, or three months, or longer. Optionally, after a period of rest, the exosome containing composition or formulation can be administered under the same or a different schedule. The period of rest can be one, two, three, or four weeks, or longer, according to the pharmacodynamic effects of the exosome containing composition or formulation on the subject. In each of these examples, the exosome containing composition or formulation may be a pharmaceutical composition or a non-pharmaceutical composition or formulation.

The exosome containing composition or formulation described herein can be administered to a subject in need thereof on a varying schedule, for example, once every four hours, every eight hours, every twelve hours, twice per day, three times per day, once per day, once every two days, once every three days, once per week, once every two weeks, once every three weeks, once every four weeks, and once per month. The duration of treatment may similarly vary in accordance with customary factors known to those of skill in the art, for example, in an acute manner, for example, dosing by administering a therapeutically effective amount of the exosomes once every two to four hours, or twice per day for 24 to 72 hours and then a different dose regimen, for example, once per day to once every two to seven days for a period of one to fourteen weeks, and all time and frequency intervals thereof.

In some embodiments, the exosome containing composition or formulation of the invention can be administered to a subject, for example a subject having experienced one or more TBI symptoms, suspected of having a TBI, or likely to develop a TBI, by parenteral administration. In these exemplary embodiments, the route of administration can be intravenous, intramuscular, subcutaneous, intracranial, intrathecal or intraarterial administration, for example, by injection. In some embodiments, therapeutically effective doses of a composition or a formulation containing MSC derived exosomes, or their internal constituents thereof can be administered intravenously, or stereotactically into the spinal cord, or the dorsal root ganglion of a subject in need thereof.

The composition of the disclosure can be delivered to the subject at a dose that is effective to treat and/or prevent one or more symptoms associated with TBI. The effective dosage will depend on many factors including the gender, age, weight, and general physical condition of the subject, the severity of the TBI, the length of time which has lapsed from the onset of symptoms, the particular compound or composition being administered, the duration of the treatment, the nature of any concurrent treatment, the carrier used, and like factors within the knowledge and expertise of those skilled in the art. As appropriate, a TBI treatment effective amount in any individual case can be determined by one of ordinary skill in the art by reference to the pertinent texts and literature and/or by using routine experimentation (see, e.g., Remington, The Science and Practice of Pharmacy (21st ed. 2005)). In one embodiment, the exosome containing composition or formulation of the invention is administered at a dose of about 0.1 to about 10.0 mg/m², e.g., about 0.6 to about 4.0 mg/m², about 1.0 to about 3.0 mg/m², or about 0.6, 0.8, 1.0, 1.2, 1.4, 1.6, 1.8, 2.0, 2.2, 2.4, 2.6, 2.8, 3.0, 3.2, 3.4, 3.6, 3.8, or 4.0 mg/m². In some instances, the dose can be even lower, e.g., as low as 0.1, 0.05, 0.01, 0.005, or 0.001 mg/m² or lower. In some instances, the dose can be even higher, e.g., as high as 20, 50, 100, 500, or 1000 mg/m² or higher. The methods of the present invention encompasses every sub-range within the cited ranges and amounts.

In some embodiments, the exosome containing composition or formulation is administered at a dose of about 1 × 10¹ to about 1 × 10¹⁵ exosomes per kg body weight of the subject, or about 1 × 10¹ to about 1 × 10¹⁴ exosomes per kg body weight of the subject, or about 1 × 10¹ to about 1 × 10¹³ exosomes per kg body weight of the subject, or about 1 × 10¹ to about 1 × 10¹² exosomes per kg body weight of the subject, or about 1 × 10¹ to about 1 × 10¹¹ exosomes per kg body weight of the subject, or about 1 × 10¹ to about 1 × 10¹⁰ exosomes per kg body weight of the subject, or about 1 × 10¹ to about 1 × 10⁹ exosomes per kg body weight of the subject, or about 1 × 10¹ to about 1 × 10⁸ exosomes per kg body weight of the subject, or about 1 × 10¹ to about 1 × 10⁷ exosomes per kg body weight of the subject, or from about 1 × 10¹ to about 1 × 10⁶ exosomes per kg body weight of the subject, or from about 1 × 10¹ to about 1 × 10⁵ exosomes per kg body weight of the subject. Preferably, the exosome containing composition or formulation of the invention is administered at a dose of about 1 × 10⁹ to about 1 × 10¹⁵ exosomes to a subject in need thereof, or a dose of about 1 × 10¹² to about 3 × 10¹² exosomes for an average adult weighing approximately 70 kg, or any relative amounts thereof for patients weighing more or less than 70 kg. The compositions for use in methods according to the present invention encompasses every sub-range within the cited ranges and amounts. In various embodiments, the exemplified doses of MSC derived exosomes per kg weight of the subject are daily doses or therapeutically effective doses, either in unit form or in sub-unit forms to be dosed one or more times per day. In all of the described methods of treatment described and illustrated above and below contemplate the use of pharmaceutically acceptable compositions comprising exosomes derived from MSCs grown in 2D cell culture or 3D cell culture, for example, 3D biopolymer scaffolds, for example, a 3D-collagen scaffold, a 3D-agarose scaffold, a 3D-matrigel scaffold, a 3D-hydrogel, a 3D-microfiber scaffold, or mixtures thereof In one embodiment the subject is one that has developed one or more symptoms of TBI after the patient has experienced a forceful contact to the skull, and the composition of the invention is administered to the subject after the development of one or more of the TBI symptoms illustrated herein. In another embodiment, the subject is one that has not developed TBI, but has had a traumatic or forceful impact to the skull and the composition of the invention is administered to the subject to prevent the occurrence of one or more TBI symptoms. In one instance, the subject is one that is undergoing an event that is likely to result in a TBI, for example, in military combat, in high impact sporting events etc. The composition of the disclosure containing exosomes (or their internal components thereof), isolated and obtained from an exosome producing cell, including without limitation, stem cells (embryonic stem cells, induced pluripotent stem cells, umbilical cord stem cells, neural stem cells, hematopoietic stem cells, e.g. MSCs, hair follicle stem cells, and other somatic stem cells) and somatic cells grown in 2D cell culture or 3D cell culture, for example, 3D biopolymer scaffolds, for example, a 3D-collagen scaffold, a 3D-agarose scaffold, a 3D-matrigel scaffold, a 3D-hydrogel, a 3D-microfiber scaffold, and mixtures thereof may be administered to the subject prior to the event occurring, concurrently with the event, and/or after the event occurs but before the development of a symptom of TBI.

The following examples of some embodiments of the invention are provided without limiting the invention to only those embodiments described herein and without disclaiming any embodiments or subject matter.

### EXAMPLES

### Abbreviations

- 2D: 2 dimension
- 3D: 3 dimension
- FBS: fetal bovine serum
- MSC: mesenchymal stromal cell
- TBI: traumatic brain injury
- DG: dentate gyrus
- EBA: endothelial barrier antigen
- NeuN: neuronal nuclei
- GFAP: glial fibrillary acidic protein
- BrdU: bromodeoxyuridine
- MWM: Morris water maze
- mNSS: modified neurological severity score
- MCID: microcomputer imaging device
- ANOVA: Analysis of variance

All experimental procedures were approved by the Henry Ford Health System Institutional Animal Care and Use Committee. To prevent potential biases of performance and detection, the persons who performed the experiments, collected data, and assessed outcome were blinded throughout the course of the experiments and were unaware of the treatment allocation.

### Example 1. Preparation of hMSCs and exosomes

hMSCs were provided by Theradigm (Bethesda, MD) and expanded, as previously described (Digirolamo et al., 1999; Qu et al., 2009). In brief, hMSCs were plated at a concentration of 3 ×10⁶ cells/75 cm² in tissue culture flasks with 20 ml low-glucose Dulbecco's modified Eagle's medium (Gibco BRL, Grand Island, NY) and were supplemented with 20% fetal bovine serum (Gibco BRL), 100 U/ml penicillin, 100 mg/ml streptomycin, and 2 mmol/L 1-glutamine. For the exosome isolation, conventional culture medium was replaced with an exosome-depleted fetal bovine serum-contained (EXO-FBS-250 A-1, System Biosciences, Mountain View, CA, USA) medium when the cells reached 60% to 80% confluence, and the hMSCs were cultured for an additional 48 hours. The media were then collected and exosomes were isolated by ExoQuick exosome isolation method according to the manufacturer's instruction. In brief, ExoQuick-TC (2.5 ml) was added to 10 ml of media, incubated 12 hours at 4°C, and centrifuged at 1500 × g for 30 min to obtain pelleted exosomes. The supernatant (non-exosomal fraction) of the samples were removed without disturbing the exosome pellets, and exosome pellets were resuspended in 200 µl of PBS. The exosomes were quantitated by measuring the total protein concentration using the micro Bicinchoninic Acid protocol (Pierce, Rockford, IL, USA) and analyzed particle size and number using a qNano nanopore-based exosome detection system according to the manufacturer's instructions (Izon, Christchurch, New Zealand).

### Example 2. Scaffold Preparation, Cell Seeding and Exosome Collection

Ultrafoam scaffolds, collagen type I, were obtained from commercial sources (Avitene Ultrafoam collagen hemostat, cat # 1050030, Davol, Cranston, RI). The samples were cut into 5 mm discs (~ 5 mm thick) under sterile conditions from the larger collagen sheets. Avitene Ultrafoam is an absorbable hemostatic sponge prepared as a sterile, porous, pliable, water insoluble partial hydrochloric acid salt of purified bovine corium collagen" The scaffolds were pre-wetted overnight at 4°C (approximately 12 h) in culture medium consisting of DMEM supplemented with 10% fetal calf serum, 100 U/mL penicillin, 100 µg/mL streptomycin, 0.1 mM non-essential amino acids, and 1 ng/mL of basic fibroblast growth factor (Life Technologies, Rockville, MD). The scaffolds were then aseptically transferred using tweezers (1 scaffold per tube) to 50-mL sterile centrifuge tubes, allowing the scaffolds to sit at the bottom of the tubes. Seeding of hMSCs on scaffolds was performed, as previously described (Lu et al., 2007).

Following trypsinization of hMSCs from ex vivo expansion conditions, hMSCs were resuspended thoroughly and transferred gently (3×10⁶ hMSCs per scaffold) into 200 µl of culture medium. Culture medium (100 µl) was then applied two times successively to opposite sides of the body of the cylindrical scaffold. The scaffold and cell solution were incubated for 30 min in a humidified incubator to facilitate primary cell seeding. The scaffolds were agitated gently within the solution manually twice every 15 min during this time. Following primary seeding, the centrifuge tubes were filled with an additional 3 mL of culture medium and placed in a humidified incubator overnight (Xiong et al., 2009). For the exosome isolation, conventional culture medium was replaced with an exosome-depleted FBS-contained medium when the cells reached 60% to 80% confluence, and the hMSCs were cultured for an additional 48 hours. The media were then collected and exosomes were isolated by ExoQuick exosome isolation method according to the manufacture's instruction, as described above.

### Example 3. Liposome Preparation

Liposomes are synthetic versions of natural vesicles such as exosomes. In order to mimic the exosomal lipid layer, we have prepared liposome based on three major fatty acids that are found in exosomal lipid analysis. Liposomes were prepared via the thin-film hydration technique (Ekanger et al., 2014). Briefly, to a 4 mL vial was added 1,2-dipalmitoyl-*sn*-glycero-3-phosphocholine (14.0 mg, 19 µmol), 1,2-distearoyl-*sn*-glycero-3-phosphocholine (4.0 mg, 5 µmol), 1,2-dioleoyl-*sn*-glycero-3-phosphocholine (4.0 mg, 5 µmol), cholesterol (8.0 mg, 2.1 µmol), and chloroform (1 mL) to produce a clear, colorless solution. Solvent was removed under reduced pressure to afford a visible film on the bottom of the vial. The hydration solution, PBS (1.15 mL) and vial containing the lipid thin film were placed in a water bath at 60 °C for 30 min, and then the hydration solution was added to the vial containing the thin film. The resulting white suspension was stirred at 60 °C for 1 h. Extrusion of the suspension was accomplished using a mini-extruder and heating block (Avanti Polar Lipids, Alabaster, AL, USA) heated to 60 °C (4 passes through a 0.2 µm polycarbonate filter followed by 15 passes through a 0.1 µm polycarbonate filter). After extrusion, the suspension was allowed to cool to ambient temperature. Liposome samples were prepared for light scattering experiments by diluting liposome suspensions in phosphate-buffered saline (PBS, 1:10, 29 mM Na2HPO4, 46 mM NaH2PO4, 57 mM NaCl, and 2.1 mM KCl). Dynamic light scattering (DLS) data were obtained using a Malvern Zetasizer Nano-ZS instrument (ZEN3600) operating with a 633 nm wavelength laser. Dust was removed from samples by filtering through 0.2 µm hydrophilic filters (Millex-LG, SLLGR04NL). The size distribution of the prepared liposome was determined by DLS and the effective diameter was about 134 nm which is in agreement with the previous report of exosomal size (Villarroya-Beltri et al., 2013).

### Example 4. Animal Model and Experimental Groups

A well-established controlled cortical impact (CCI) rat model of TBI was utilized for the present study (Dixon et al., 1991). Adult male Wistar rats weighing 317 ± 10 g (2-3 months old) were anesthetized with chloral hydrate (350 mg/kg body weight, intraperitoneally). Rectal temperature was maintained at 37 ± 5°C using a feedback-regulated water-heating pad. Rats were placed in a stereotactic frame. Two 10-mm-diameter craniotomies were performed adjacent to the central suture, midway between Lambda and Bregma. The second craniotomy allowed for lateral movement of cortical tissue. The dura mater was kept intact over the cortex. Cortical injury was delivered by impacting the left cortex (ipsilateral cortex) with a pneumatic piston containing a 6-mm-diameter tip at a rate of 4 m/s and 2.5 mm of compression. Velocity was measured with a linear velocity displacement transducer.

The study animals were randomly divided into the following groups (n = 8/group): 1) Sham (craniotomies without injury and treatment), 2) TBI + liposomes (Lipo), 3) TBI + exosomes from hMSCs in 2D culture (Exo-2D), 4) TBI+ exosomes from hMSCs in 3D culture (Exo-3D). Exosomes generated from hMSCs in 2D or 3D conditions (100 µg total protein of exosome precipitate in 0.5 ml PBS/rat, ~=3×10⁹ particles) or an equal volume of PBS containing 3×10⁹ liposomes (0.5 ml) was administered intravenously over 5 min via tail vein, starting 1 day after injury. The dose of exosomes was chosen based on previous studies with exosomes in rats after stroke (Xin et al., 2013b) and TBI (Zhang et al., 2015). TBI animals treated with liposomes were used as a control group. Liposomes (the liposome mimic of exosome lipid contents) we administered have the same lipid components as exosomes but lack content of proteins and genetic materials. We generated liposomes mimicking the primary content of the exosome lipid/phospholipid content. We performed the control treatment with the artificial exosome membrane consisting of the same lipid content as exosomes to tease out potential therapeutic effects of exosomes in part due to their structural components. Sham animals underwent surgery without injury and treatment. For labeling proliferating cells, 5-bromo-2'-deoxyuridine (BrdU, 100 mg/kg) was injected intraperitoneally into rats daily for 10 days, starting 1 day after TBI. The dose and time for BrdU injection was based on our previous TBI studies in rats (Xiong et al., 2011a). All animals were allowed to survive 35 days after TBI.

### Example 5. Evaluation of Neurological Outcome

### Modified Neurological Severity Score (mNSS) Test

Neurological functional measurement was performed using the mNSS test (Chen et al., 2001b). The test was carried out on all rats prior to injury and at 1, 4, 7, 14, 21, 28 and 35 days after TBI. The mNSS is a composite of the motor (muscle status, abnormal movement), sensory (visual, tactile, and proprioceptive), and reflex tests and has been used in previous studies (Lu et al., 2007; Mahmood et al., 2014d). Neurological function was graded on a scale of 0 to 18 (normal score 0; maximal deficit score 18). In the severity scores of injury, one point is awarded for each abnormal behavior or for lack of a tested reflex; thus, the higher the score, the more severe the injury.

### Example 6. Foot fault Test

To evaluate sensorimotor function, the foot fault test was carried out before TBI and at 1, 4, 7, 14, 21, 28 and 35 days after TBI. The rats were allowed to walk on a grid. With each weight-bearing step, a paw might fall or slip between the wires and, if this occurred, it was recorded as a foot fault (Baskin et al., 2003). A total of 50 steps were recorded for the right forelimb.

### Example 7. Morris Water Maze (MWM) Test

To measure spatial learning impairments, an updated version of the MWM test was used (Choi et al., 2006). The procedure was modified from previous versions (Morris et al., 1982), and has been used for spatial memory assessment in rodents with brain injury (Choi et al., 2006). The MWM test was performed monthly postinjury. At each testing interval, animals were tested with 4 trials per day for 5 consecutive days on Day 31-35 after TBI. A blue swimming pool (1.8 m in diameter) was located in a large room, where there were many clues external to the maze (e.g., pictures on the walls, lamps and a camera on the ceiling); these were visible from the pool and presumably used by the rats for spatial orientation. The position of the cues remained unchanged throughout the experiment. Data collection was automated using the Human Visual Image (HVS) Image 2020 Plus Tracking System (US HVS Image, San Diego, CA), as described previously (Mahmood et al., 2007). For data collection, the swimming pool was subdivided into four equal quadrants formed by imaging lines. At the start of each trial, the rat was placed at one of four fixed starting points, randomly facing toward a wall (designated North, South, East and West) and allowed to swim for 90 seconds or until it found the platform which was transparent and invisible to animals. If the animal found the platform by spatial navigation, it was allowed to remain on it for 10 seconds. Throughout the test period, the platform was located in the northeast (NE) quadrant 2 cm below water in a randomly changing position, including locations against the wall, toward the middle of the pool, or off-center but always within the target quadrant. If the animal was unable to locate the platform within 90 seconds, the trial was terminated after being guided onto the platform and remaining on it for 10 seconds, and a maximum score of 90 seconds was assigned. The percentage of time the animals spent swimming within the target quadrant relative to the total amount of time swimming in the maze before reaching the hidden platform or within 90 seconds for those rats that failed to find the platform was recorded for statistical analysis. The latency to find the hidden escape platform was also recorded and analyzed. In the traditional version of the MWM test, the position of the hidden platform is always fixed and is relatively easy for rodents. With the modified MWM test we used in this study, the platform is relocated randomly within the correct quadrant with each training trial. The rodents must spend more time searching within the target quadrant; therefore each trial effectively acts as a probe trial. The advantage of this protocol is that rodents should find the platform purely and extensively by reference to the extra-maze spatial cues, which improves the accuracy of spatial performance in the MWM (Choi et al., 2006).

### Example 8. Tissue Preparation

Rats were anesthetized with chloral hydrate administered intraperitoneally and perfused transcardially with saline solution, followed by 4% paraformaldehyde in 0.1 M PBS, pH 7.4. Rat brains were removed and immersed in 4% paraformaldehyde for 2-4 days. Using a rat brain matrix (Activational Systems Inc.), each forebrain was cut into 2- mm thick coronal blocks for a total 7 blocks from Bregma 5.2 mm to Bregma -8.8 mm per animal (Paxinos and Watson, 1986). The tissues were embedded in paraffin and a series of 6 (µm-thick slides were cut. For lesion volume measurement, one 6-µm-thick section from each of 7 coronal blocks was traced by a microcomputer imaging device (MCID) (Imaging Research, St. Catharine's, Ontario, Canada), as described previously (Chen et al., 2005). The volumes of the ipsilateral and contralateral cortices were computed by integrating the area of each cortex measured at each coronal level and the distance between two sections. The cortical lesion volume was expressed as a percentage calculated by [(contralateral cortical volume - ipsilateral cortical volume)/(contralateral cortical volume) × 100% (Swanson et al., 1990).

### Example 9. Immunohistochemistry

Antigen retrieval was performed by boiling brain sections in 10 mM citrate buffer (pH 6.0) for 10 minutes. After washing with PBS, sections were incubated with 0.3 % H₂O₂ in PBS for 10 minutes, blocked with 1% BSA containing 0.3 % Triton-X 100 at room temperature for 1 hour, and incubated with anti-endothelial barrier antigen (EBA, 1:1000; COVANCE, CA) or anti-CD68 (1:200; Serotec, Kidlington, UK) or anti-glial fibrillary acidic protein (GFAP, 1:1000; Dako, Denmark) at 4°C overnight. For negative controls, primary antibodies were omitted. After washing, sections were incubated with biotinylated anti-mouse or anti-rabbit antibodies (1:200; Vector Laboratories, Inc.) at room temperature for 30 minutes. After an additional washing, sections were incubated with an avidin-biotin-peroxidase system (ABC kit, Vector Laboratories, Inc.), visualized with diaminobenzidine (Sigma), and counterstained with hematoxylin.

### Example 10. Immunofluorescent Staining

Newly generated endothelial cells and newborn mature neurons in the lesion boundary zone and dentate gyrus 35 days after TBI were identified by double labeling for BrdU with EBA or neuronal nuclei (NeuN), respectively. In brief, after being deparaffinized and rehydrated, brain sections were boiled in 10 mM citric acid buffer (pH 6) for 10 minutes. After washing with PBS, sections were incubated in 2.4 N HCl at 37°C for 20 minutes. Sections were incubated with 1% BSA containing 0.3% Triton-X-100 in PBS. Sections were then incubated with mouse anti-NeuN antibody (1:200; Chemicon, Temecula, CA) or anti-EBA at 4°C overnight. For negative controls, primary antibodies were omitted. FITC-conjugated anti-mouse antibody (1:400; Jackson ImmunoResearch, West Grove, PA) was added to sections at room temperature for 2 hours. Sections were then incubated with rat anti-BrdU antibody (1:200; Dako, Glostrup, Denmark) at 4°C overnight. Sections were then incubated with Cy3-conjugated goat anti-rat antibody (1:400; Jackson ImmunoResearch, West Grove, PA) at room temperature for 2 hours. Each of the steps was followed by three 5-minute rinses in PBS. Tissue sections were mounted with Vectashield mounting medium (Vector laboratories, Burlingame, CA).

### Example 11. Cell Counting and Quantitation

CD68+ microglia/macrophages, EBA+ endothelial cells, GFAP+ astrocytes, BrdU+ cells, and EBA/BrdU-colabeled cells were counted in the lesion boundary zone and the dentate gyrus (DG). For analysis of neurogenesis, we counted NeuN/BrdU-colabeled cells in the DG and its subregions, including the subgranular zone, granular cell layer, and the molecular layer. The fields of interest were digitized under the light microscope (Nikon, Eclipse 80i) at a magnification of either 200 or 400 using CoolSNAP color camera (Photometrics) interfaced with MetaMorph image analysis system (Molecular Devices), as described in detail previously (Zhang et al., 2013b). In brief, five fields of view in the lesion boundary zone from the epicenter of the injury cavity (Bregma -3.3 mm), and 9 fields of view in the ipsilateral DG were counted in the each section. From our previous experience, our inter-rater reliability was greater than 95% when the cell counts were compared between two independent trained blinded observers scoring the same sections of an animal. In the present study, one blinded observer performed the cell counting in all brain sections.

### Statistical Analysis

Data are presented as the means with standard deviations. The analysis of variance (ANOVA) was used for repeated measurements of spatial performance and sensorimotor function. For cell counting, a one-way ANOVA followed by post hoc Tukey's tests was used to compare the differences between the exosome-treated, liposome-treated and sham groups. Pearson's correlation coefficients were calculated to examine relationships between cognitive functional recovery and immunostaining. Differences were considered significant if the P value was <0.05.

### Example 12. Results

Isolation of exosomes from hMSCs cultured in 2D and 3D conditions.

In the present disclosure, the ExoQuick-TC kit was employed with centrifugation to isolate exosomes (see the methods described in Taylor et al., 2011). Due to the relatively low centrifugal force employed in the ExoQuick isolation process, the precipitation of non-exosomal proteins and nucleotides is minimized, whereas non-exosomal protein contamination can occur in prolonged ultra-centrifugation methods (Cvjetkovic et al., 2014). The protein amount of exosomes generated from 3 × 10⁶ hMSCs in the 3D condition was 2 times higher than that of those in the 2D condition (168.5 ± 11.2 µg for 3D vs 84.7 ± 5.5 µg for 2D, p<0.05). Exosomes were isolated from culture media where 3 × 10⁶ hMSCs were cultured in 2D dish or 3D scaffold in the exosome-depleted FBS-contained medium for 2 days.

### Example 13. hMSC Exosome Administration Does Not Alter Cortical Lesion Volume in Rats after TBI

Cortical lesion volume was measured 35 days post TBI, as described previously (Xiong et al., 2011b). No differences in the lesion volume were observed between the liposome group and exosome groups (16.4 ± 0.2% for Lipo group, 16.2 ± 0.4% for Exo-2D group, 16.0 ± 0.3% for Exo-3D group, Fig.1, p > 0.05).

### Example 14. hMSC Exosome Administration Significantly Promotes Sensorimotor Functional Recovery in Rats after TBI

Neurological functional measurement was performed on rats using the mNSS test. The higher the score, the more severe the injury. The mNSS score was close to 12 in TBI rats (both the Lipo and exosome groups) on Day 1 post TBI, indicating neurological functional deficits were comparable in all TBI rats before treatment (Fig. 2A, p > 0.05). Significant reduction in the mNSS score was found over time in the liposome-treated animals starting from Day 4-35 compared to Day 1 post injury (p < 0.05), suggesting a significant spontaneous sensorimotor functional recovery occurred after TBI. However, compared to the liposome treatment, functional recovery was significantly improved in the exosome-treated groups on Days 14-35 after TBI (at Day 14-35, p < 0.05, with ANOVA followed by post-hoc Tukey's tests). Exosome treatment also significantly reduced the frequency of forelimb foot fault occurrence as compared to liposome controls (Fig. 2B, at Day 14-35, p < 0.05, with ANOVA followed by post-hoc Tukey's tests). Although both Exo-2D and Exo-3D treatments significantly improved sensorimotor functional recovery compared to the liposome treatment, there is no significant difference in mNSS score and foot fault test between the Exo-2D and Exo-3D groups.

### Example 15. hMSC Exosome Administration Significantly Enhances Spatial Learning in Rats after TBI

Spatial learning measurements were performed during the last five days (31-35 days post injury) prior to sacrifice using a modified MWM test, which is very sensitive to the hippocampal injury (Choi et al., 2006). The greater the percentage of time the animals spend in the correct quadrant (i.e., Northeast, where the hidden platform was located) in the water maze, the better the spatial learning function. The percentage of time spent by sham rats in the correct quadrant increased significantly from 32-35 days after sham operation, compared to time spent in the correct quadrant at the first day of testing, that is, Day 31 (Fig. 3A, p < 0.05). In the testing of spatial memory among 3 groups, no significant between-group effect on the time spent in the correct quadrant was detected on the first day of the testing in the MWM test (Day 31 post injury, p > 0.05); however, a statistically significant between-group effect on the time spent in the correct quadrant was noted in the MWM test (at Day 33-35, p < 0.05). Relative to the liposome group, post-hoc Tukey's testing demonstrated significantly increased time spent in the correct quadrant in the exosome groups at Day 33-35 with significantly improved benefits from Exo-3D treatment compared with the Exo-2D treatment (p < 0.05).

Another important parameter for assessing spatial learning in the MWM test is the time (referred as latency) for animals to find the hidden platform in the correct quadrant (Zhang et al., 2013b). The less time for animals to find the platform, the better the spatial learning function. During the first day of testing (that is, 31 days post injury), no significant between-group effect on the time for animals to find the hidden platform in the correct quadrant was detected in the MWM test (Fig. 3B, p = 0.338); however, sham animals took significantly less time to find the hidden platform in the correct quadrant compared to other groups during the second day of testing (at Day 32, p < 0.05). Relative to the liposome group, post-hoc Tukey's testing demonstrated significantly less time for animals to find the platform in the exosome groups during the last 3 day testing, that is, at Day 33-35 (p < 0.05). Relative to the Exo-2D group, animals in the Exo-3D group took significantly less time to find the hidden platform at Day 33-35 (p < 0.05).

### Example 16. hMSC Exosome Administration Significantly Increases Vascular Density and Angiogenesis in Rats after TBI

EBA-staining was performed to identify mature vasculature in the brain after TBI (Li et al., 2007). TBI alone significantly increased the density of vessels in the lesion boundary zone and DG of the ipsilateral hemisphere compared to sham controls (Fig. 4, p < 0.05). Exosome treatments significantly increased the vascular density in the injured cortex and DG compared to the liposome treatment (Fig. 4, p < 0.05, with ANOVA followed by post-hoc Tukey's tests). Exosome treatment significantly increased angiogenesis identified by EBA/BrdU+ double labeling for newborn endothelial cells in the lesion boundary zone and DG compared to the liposome treatment (Fig. 4, p < 0.05). The Pearson's correlation analyses further showed that: 1) spatial learning was positively correlated to EBA+ vascular density in the DG region (R² = 0.87, p < 0.05); and 2) sensorimotor functional recovery was positively correlated to EBA+ vascular density in the lesion boundary zone (R² = 0.75, p < 0.05). Although both Exo-2D and Exo-3D treatments significantly promoted angiogenesis compared to the liposome treatment, there is no significant difference between the Exo-2D and Exo-3D groups.

### Example 17. hMSC Exosome Administration Significantly Increases Neurogenesis in the DG in Rats after TBI

To investigate cell proliferation in the DG, we injected BrdU ip into rats once daily for 10 days starting 24 hours post injury. Animals were sacrificed at 35 days after TBI, and immunostaining were performed on paraffin-embedded brain coronal sections (Meng et al., 2011). TBI alone significantly increased cell proliferation compared to Sham group. Exosome therapy did not significantly increase the number of BrdU-positive cells compared to the liposome treatment (Fig. 5A-D, p < 0.05). To identify newly generated neurons in the DG, double staining for BrdU (proliferating marker) and NeuN (mature neuronal marker) was performed. Exosome treatment significantly increased the number of newborn neurons detected in the granule layer of the DG compared to the liposome controls (Fig. 5M-O, p < 0.05). Relative to the Exo-2D treatment, the Exo-3D treatment significantly increased the number of newborn mature neurons detected in the DG (p < 0.05). Our data show a significant positive correlation between spatial learning tested by the MWM test and the number of newborn mature neurons in the DG (R² = 0.73, p< 0.05).

### Example 18. hMSC Exosome Administration Significantly Reduces Brain Inflammation in Rats after TBI

GFAP-staining was performed to identify reactive astrocytes in the brain after TBI (Schwab et al., 2001). TBI alone significantly increased the number of GFAP+ cells in the lesion boundary zone and DG of the ipsilateral hemisphere compared to sham controls (Fig. 6, p < 0.05). Exosome treatment significantly reduced the GFAP+ astrocyte density in the injured cortex and DG compared to the liposome treatment (Fig. 6, p < 0.05, with ANOVA followed by post-hoc Tukey's tests). The Pearson's correlation analyses showed that: 1) spatial learning was inversely correlated to GFAP+ astrocyte density in the DG region (R² = 0.86, p < 0.05); and 2) sensorimotor functional recovery was inversely correlated to GFAP+ astrocyte density in the lesion boundary zone (R² = 0.77, p < 0.05). CD68-staining was performed to identify activated macrophages/microglia in the brain after TBI (Li et al., 2009). TBI alone significantly increased the number of CD68+ cells in the lesion boundary zone and DG of the ipsilateral hemisphere compared to sham controls (Fig. 6, p < 0.05). Exosome treatments significantly reduced the CD68+ cell number in the injured cortex and DG compared to the liposome treatment (Fig. 6, p < 0.05, with ANOVA followed by post-hoc Tukey's tests). Relative to the Exo-2D treatment, the Exo-3D treatment significantly decreased the number of CD68+ cells detected in the DG (p < 0.05). The Pearson's correlation analyses showed that: 1) spatial learning was inversely correlated to CD68+ cell density in the DG region (R² = 0.75, p < 0.05); and 2) sensorimotor functional recovery was inversely correlated to CD68+ cell density in the lesion boundary zone (R² = 0.55, p < 0.05).

### Example 19. Discussion

In the present study, the inventors have demonstrated for the first time that systemic administration of cell-free exosomes generated by human MSCs cultured under 2D and 3D conditions, with treatment initiated 24 hours post injury in a rat model of TBI does not alter cortical lesion volume compared to the liposome treatment control, but significantly: 1) improves cognitive and sensorimotor functional recovery; 2) increases the number of newborn mature neurons in the DG; and 3) increases the number of newborn endothelial cells in the lesion boundary zone and DG; 4) reduces neuroinflammation; and 5) exosomes generated from hMSCs cultured in 3D condition provide better outcome in spatial learning compared to exosomes from 2D culture. Exosome treatments initiated 24 h post injury did not reduce lesion volume, suggesting that beneficial effects of exosomes is not attributed to direct neuroprotection, but, rather to neurovascular remodeling. Improved functional recovery after treatment of TBI with exosomes generated from human MSCs is significantly associated with increased brain angiogenesis and neurogenesis as well as with reduced neuroinflammation. Our results suggest that intravenous administration of exosomes generated from human MSCs may represent a novel cell-free therapeutic approach for treatment of TBI.

MSCs alone and MSCs seeded into scaffolds improve functional outcome in animal models of stroke and TBI (Kim et al., 2010; Lu et al., 2007; Mahmood et al., 2004a; Mahmood et al., 2005, 2007; Mahmood et al., 2014a; Mahmood et al., 2014d; Peng et al., 2015; Qu et al., 2011; Qu et al., 2008; Xiong et al., 2009). The mechanisms underlying improvement in MSC-induced functional recovery after TBI are not clear. A recent study shows that intravenous administration of cell-free MSC-generated exosomes improves functional recovery and enhances neurite remodeling, neurogenesis, and angiogenesis in rats after stroke (Xin et al., 2013b). Cells produce exosomes with components and functions that mirror those of their parent cells (Katsuda et al., 2013). Exosomes contain proteins, lipids, messenger RNAs and microRNAs, which can be transferred to recipient cells and modify their characteristics (Xu et al., 2013). Selective manipulation of specific molecules identified for a therapeutic effect in the parent MSCs may lead to an enhancement of the therapeutic efficiency of isolated exosomes, as demonstrated in our previous stroke study showing that exosomes from MSCs mediate the miR-133b transfer to astrocytes and neurons, which regulate gene expression, and subsequently increase neurite remodeling and functional recovery after stroke (Xin et al., 2013g). Further studies are warranted to identify the molecular constituents of the exosomes derived from human MSCs, including specific microRNAs and growth factors that promote angiogenesis and neurogenesis as well as reduce neuroinflammation after TBI.

A clear distinction between the endosomal origin exosomes (30-120 nm in diameter) and microvesicles is lacking , and it is technically difficult to definitively separate microvesicles from the culture media by currently available methods like ultracentrifugation, density gradient separation, chromatography and immunoaffinity capture methods (Lotvall et al., 2014; Tauro et al., 2012). Exosomes accumulate as intraluminal vesicles inside multivesicular bodies and are released after fusion with the plasma membrane (Denzer et al., 2000; Gruenberg et al., 1989; Stoorvogel et al., 2002; van Doormaal et al., 2009) while microvesicles (size 100~1000 nm) are small, plasma-membrane-derived particles and are released into the extracellular environment by the outward budding and fission of the plasma membrane (Amano et al., 2001; Cocucci et al., 2009; Muralidharan-Chari et al., 2010). In the present disclosure, the 100 µg total protein of exosomes injected into each rat was collected from approximately 2 × 10⁶ MSCs, a number of MSCs equivalent to the effective amount that the inventors previously used in the MSC-based treatment for TBI (2 × 10⁶ MSCs per rat) (Lu et al., 2001c). However, our previous studies indicate that only a small percentage (<1 %) of transplanted MSCs via tail vein injection can be detected in the injured brain (Mahmood et al., 2001). Although our recent study using exosomes tagged with GFP demonstrated that exosome-enriched extracellular particles were released from MSCs intravenously administered to stroke rats and transferred to adjacent astrocytes and neurons (Xin et al., 2013g), the quantity of exosomes generated by the transplanted MSCs in the brain after intravenous MSC administration is not known. The present invention demonstrates that human MSCs seeded in the 3D collagen scaffolds generated significantly more exosomes compared to the human MSCs cultured in the 2D condition. The 3D collagen scaffold also unexpectedly generated exosomes containing a distinct set of proteins and nucleic acids as those contained in exosomes generated from 2D cell culture conditions. Doses of exosomes can be administered to a subject, for example a human. Without being bound to any particular theory, it is believed that exosomes may act, at least in part, when administered in the form of cell-based therapies, on peripheral tissues to indirectly promote neurovascular remodeling and functional recovery post TBI. MSCs used as cell therapy after TBI may act as remote "bioreactors" via stimulation of lung macrophages and spleen T regulatory cell production (likely due to many intravenously injected MSCs trapped by these organs), leading to systemic remote effects on the central nervous system (Walker et al., 2012).

In previous studies, tagging exosomes with green fluorescent protein (GFP), the inventors demonstrated that exosomes-enriched particles generated from MSCs were taken up by astrocytes and neurons in the ischemic boundary zone in stroke rats after intravenous injection of MSCs (Xin et al., 2013g). Pharmacokinetic analysis revealed that exosomes derived from B16-BL6 murine melanoma cells disappeared very quickly from the blood circulation with a half-life of approximately 2 min after intravenous injection (Takahashi et al., 2013). The injected exosomes were detected mainly in the liver, spleen, and lung at 4 h after iv injection, and the liver was the major organ in the clearance of exogenously administered B16BL6-derived exosomes (Morishita et al., 2015). Macrophages in the liver and spleen play important roles in the clearance of intravenously injected exosomes from the systemic circulation (Imai et al., 2015). TBI causes the opening/damage of the BBB (Thai and Neuhaus, 2014) which may facilitate exosomes to enter the brain.

Exosomes contain very complex molecular cargo (Lai et al., 2013; Yu et al., 2014). The benefit and potential strength of exosome treatment, as with stem-cell therapy, is its ability to affect multiple targets. The inventors have previously demonstrated in stroke rats, that treatment with MSCs transfers microRNAs via exosomes to recipient parenchymal cells (Xin et al., 2013g). MicroRNAs regulate a myriad of genes (Lakshmipathy and Hart, 2008). It is likely that the multitargeted approach, rather than the traditional, single molecular pathway approach, elicits the therapeutic potency of exosomes or cell-based therapy. Treatment with MSC-generated exosomes is an alternative approach for targeting the complex TBI.

The experimental data provided in the present disclosure demonstrates that intravenous administration of exosomes derived from human MSCs cultured in 2D and 3D conditions promotes neurogenesis in the DG after TBI in an animal *in vivo* model. Neurogenesis (a complex process by which new neurons are generated from neural stem/progenitor cells during development) occurs in mammals during adulthood, and is involved in the pathology of different neurological disorders (Taupin, 2006). Thus, neurogenesis is a potential target for treatment of neurological diseases. TBI stimulates neurogenesis in rodents and humans (Kernie and Parent, 2010; Richardson et al., 2007; Zheng et al., 2013). There is a strong link between certain types of memory functions and adult neurogenesis in the hippocampus. For example, blocking neurogenesis genetically (Blaiss et al., 2011) or pharmaceutically (Zhang et al., 2012) impairs spatial learning and memory after TBI, while enhancing neurogenesis promotes learning and memory (Kleindienst et al., 2005; Lu et al., 2005; Sun et al., 2007). Adult neurogenesis in the mammalian brain occurs primarily in the DG of the hippocampus and in the subventricular zone (SVZ) surrounding the lateral ventricle (Altman and Das, 1965; Lois and Alvarez-Buylla, 1993). In normal conditions, neuroblasts in the SVZ migrate along the rostral migratory stream to the olfactory bulb and differentiate into olfactory interneurons (Doetsch and Alvarez-Buylla, 1996). Some of neuroblasts generated in the SVZ can migrate to the injured cortical area after TBI and may be involved in brain injury repair (Jin et al., 2003; Parent et al., 2002; Sundholm-Peters et al., 2005). To date, there is no evidence for migration of SGZ-derived cells beyond the hippocampus after brain injury. In the DG, the newly generated cells of the subgranular zone (SGZ) migrate laterally into the granule cell layer and exhibit properties of fully integrated mature dentate granule neurons (Kempermann and Gage, 2000; van Praag et al., 2002b). Importantly, the newly generated DG granule neurons form synapses and extend axons into their correct target area, the CA3 region (Hastings and Gould, 1999).

The data provided herein indicates that human MSC-derived exosome treatment enhances generation of newly born vessels (angiogenesis), which may contribute to functional recovery after TBI. EBA+ cells are endothelial cells which constitute the vessels (Lin et al., 2001). Exosome treatment-induced angiogenesis may contribute motor functional recovery by promoting neurite growth and synaptogenesis in the brain after stroke (Xin et al., 2013b) because angiogenesis is well coupled with neurogenesis in the DG (Arai et al., 2009; Lo, 2008; Ohab et al., 2006; Xiong et al., 2011c). These neurovascular coupling and remodeling may act, in concert, to improve learning and memory after brain injury.

In the present disclosure, experimental results indicate that cell-free exosomes derived from human MSCs promote neurovascular remodeling and improve functional recovery after TBI, which supports the inventor's findings indicating that the efficacy of MSC transplantation in treating TBI in animal models is independent of cell replacement (Chopp et al., 2008; Joyce et al., 2010). The inventors have previously employed different routes (intraarterial, intravenous, and intracerebral) to administer MSCs into rodents with TBI (Lu et al., 2001a; Mahmood et al., 2004a; Mahmood et al., 2002). Although they exhibit promising therapeutic effects (Lu et al., 2001a; Mahmood et al., 2008; Mahmood et al., 2004a; Mahmood et al., 2002), there are some disadvantages for each route. Relatively few MSCs can be injected intracranially; intraarterial injection of MSCs can cause brain ischemia; and intravenous injection results in body-wide distribution of MSCs (Lu et al., 2007). Although transplantation of scaffolds seeded with MSCs into lesion cavity promotes functional recovery after TBI (Lu et al., 2007; Mahmood et al., 2011; Mahmood et al., 2014d; Xiong et al., 2009), it requires cranial surgery. Considering the nanosize of exosomes and their many advantages, exosomes present a novel weapon for the treatment of TBI in terms of easy administration and the potential drug delivery vehicles across the BBB (Alvarez-Erviti et al., 2011; Braccioli et al., 2014). Although cell-free exosome-based therapy offers several advantages over MSCs including easier storage and reduced safety risks.

In the results and data provided herewith, activation of GFAP+ astrocytes and CD68+ microglia/macrophages was significantly suppressed by exosomes compared to the liposome treatment control. This anti-inflammatory effect is observed when MSCs are administered as a therapeutic treatment in animal models of stroke (Tsai et al., 2014; Xin et al., 2013a) and TBI (Zhang et al., 2013a). Astrocytes and microglia are distributed throughout the brain, and one of their main functions is to monitor and sustain neuronal health (Woodcock and Morganti-Kossmann, 2013). Activated astrocytes and microglia release pro and anti-inflammatory cytokines, free radicals, anti-oxidants, and neurotrophic factors which contribute to neuronal death as well as enhance survival mechanisms during neurodegeneration (Singh et al., 2011) and after TBI (Li et al., 2009; Zhang et al., 2012). Recent experimental evidence indicates that microglia under certain circumstances can be beneficial and support the different steps in neurogenesis, progenitor proliferation, survival, migration, and differentiation (Ekdahl et al., 2009). In the present disclosure, experimental data suggest that suppression of activated microglia/macrophages by treatment with exosomes may, at least in part, contribute to increased angiogenesis and neurogenesis, and subsequent improvement in functional recovery after TBI. The unexpected finding of improved functional outcome in spatial learning from TBI rats treated with exosomes isolated from human MSCs cultured in 3Dmay be attributed to further enhanced neurogenesis and reduced activation of microglia/astrocytes in the DG compared to the exosomes isolated from human MSCs cultured in 2D.

In the present experimental *in vivo* 35-day study, the exosome treatment significantly accelerated functional recovery (that is, reduced mNSS and foot fault scores) after incurring TBI compared to the control liposome treatment. TBI produces behavioral deficits, with different recovery rates over time, dependent on injury type, severity/size, sex, age, and different tasks performed (Ding et al., 2013; Ning et al., 2011; Nishibe et al., 2010; Smith et al., 2007). Our previous long-term (3-month) studies indicate that TBI animals without interventions continue to slowly recover after the 35 day time point (Mahmood et al., 2008; Ning et al., 2011). The results and data obtained herewith provides evidence that exosome, and particularly 3D scaffold generated exosomes are beneficial for treatment of TBI.

Exosomes, depending on their parental origin, contain a variety of proteins, lipids, noncoding RNAs, mRNA, and miRNA, collectively termed as "cargo" contents (Kalani et al., 2014). Contained among these constituents, miRNAs may play a key role in mediating biological function due to their prominent role in gene regulation, as we demonstrate that MSCs communicate with brain parenchymal cells and regulate neurite outgrowth by transfer of miR-133b to neural cells via exosomes (Chopp and Zhang, 2015; Xin et al., 2012; Xin et al., 2014; Xin et al., 2013g; Zhang and Chopp, 2015). In addition, we performed the control treatment with the artificial exosome membrane, which consists of the same lipid content as exosomes but lacks content of proteins and genetic materials. We demonstrate that it is the content of the exosome and not the lipid membrane of the exosome that mediates functional recovery. We have discovered a new way to enhance production of exosomes via 3D matrices.

The inventors have also unexpectedly discovered that MSCs grown in 3D biopolymer scaffolds, (for example, a 3D-collagen scaffold, a 3D-agarose scaffold, a 3D-matrigel scaffold, a 3D-hydrogel, a 3D-microfiber scaffold, and mixtures thereof), produce a greater quantity of exosomes per cell and exosomes containing a different set of proteins and nucleic acids that were found to significantly improving functional recovery and promoting neurovascular remodeling as well as reducing neuroinflammation in comparison to exosomes derived from MSCs grown and cultured in 2D. The findings of the present data is believed to be extrapolated to other exosome producing cells, for example, stem cells (embryonic stem cells, induced pluripotent stem cells, umbilical cord stem cells, neural stem cells, hematopoietic stem cells, hair follicle stem cells, and other somatic stem cells) and somatic cells, (for example, fibroblasts, Schwann cells, microglia, lymphocytes, dendritic cells, mast cells, and endothelial cells).

In conclusion, the present disclosure and examples provide to the best of the inventor's knowledge, demonstration for the first time that intravenous administration of exosomes generated from exosome producing cells, such as human MSCs grown in 2D or 3D cultures improves functional recovery and promotes neurovascular remodeling (angiogenesis and neurogenesis) and reduces neuroinflammation in rats after TBI. Exosomes generated from human MSCs grown in 2D or 3D cultures have been shown to improve functional recovery after TBI. Cell-free exosomes may represent a novel and safer therapeutic refinement of MSCs for treatment of TBI and other neurological diseases.

### References

Akyurekli, C., Le, Y., Richardson, R.B., Fergusson, D., Tay, J., Allan, D.S., 2015. A systematic review of preclinical studies on the therapeutic potential of mesenchymal stromal cell-derived microvesicles. Stem Cell Rev 11, 150-160.
Alvarez-Erviti, L., Seow, Y., Yin, H., Betts, C., Lakhal, S., Wood, M.J., 2011. Delivery of siRNA to the mouse brain by systemic injection of targeted exosomes. Nat Biotechnol 29, 341-345.
Amano, T., Furuno, T., Hirashima, N., Ohyama, N., Nakanishi, M., 2001. Dynamics of intracellular granules with CD63-GFP in rat basophilic leukemia cells. J Biochem 129, 739-744.
Arai, K., Jin, G., Navaratna, D., Lo, E.H., 2009. Brain angiogenesis in developmental and pathological processes: neurovascular injury and angiogenic recovery after stroke. FEBS J 276, 4644-4652.
Barteneva, N.S., Maltsev, N., Vorobjev, I.A., 2013. Microvesicles and intercellular communication in the context of parasitism. Front Cell Infect Microbiol 3, 49.
Baskin, Y.K., Dietrich, W.D., Green, E.J., 2003. Two effective behavioral tasks for evaluating sensorimotor dysfunction following traumatic brain injury in mice. J Neurosci Methods 129, 87-93.
Blaiss, C.A., Yu, T.S., Zhang, G., Chen, J., Dimchev, G., Parada, L.F., Powell, C.M., Kernie, S.G., 2011. Temporally specified genetic ablation of neurogenesis impairs cognitive recovery after traumatic brain injury. J Neurosci 31, 4906-4916.
Borges, F.T., Reis, L.A., Schor, N., 2013. Extracellular vesicles: structure, function, and potential clinical uses in renal diseases. Braz J Med Biol Res 46, 824-830.
Braccioli, L., van Velthoven, C., Heijnen, C.J., 2014. Exosomes: a new weapon to treat the central nervous system. Mol Neurobiol 49, 113-119.
Chen, J., Li, Y., Wang, L., Zhang, Z., Lu, D., Lu, M., Chopp, M., 2001a. Therapeutic benefit of intravenous administration of bone marrow stromal cells after cerebral ischemia in rats. Stroke 32, 1005-1011.
Chen, J., Sanberg, P.R., Li, Y., Wang, L., Lu, M., Willing, A.E., Sanchez-Ramos, J., Chopp, M., 2001b. Intravenous administration of human umbilical cord blood reduces behavioral deficits after stroke in rats. Stroke 32, 2682-2688.
Chen, J., Zhang, C., Jiang, H., Li, Y., Zhang, L., Robin, A., Katakowski, M., Lu, M., Chopp, M., 2005. Atorvastatin induction of VEGF and BDNF promotes brain plasticity after stroke in mice. J Cereb Blood Flow Metab 25, 281-290.
Chen, J., Zhang, Z.G., Li, Y., Wang, L., Xu, Y.X., Gautam, S.C., Lu, M., Zhu, Z., Chopp, M., 2003. Intravenous administration of human bone marrow stromal cells induces angiogenesis in the ischemic boundary zone after stroke in rats. Circ Res 92, 692-699.
Choi, S.H., Woodlee, M.T., Hong, J.J., Schallert, T., 2006. A simple modification of the water maze test to enhance daily detection of spatial memory in rats and mice. J Neurosci Methods 156, 182-193.
Chopp, M., Li, Y., 2002. Treatment of neural injury with marrow stromal cells. Lancet Neurol 1, 92-100.
Chopp, M., Li, Y., Zhang, J., 2008. Plasticity and remodeling of brain. J Neurol Sci 265, 97-101.
Chopp, M., Zhang, Z.G., 2015. Emerging potential of exosomes and noncoding microRNAs for the treatment of neurological injury/diseases. Expert Opin Emerg Drugs, 1-4.
Cocucci, E., Racchetti, G., Meldolesi, J., 2009. Shedding microvesicles: artefacts no more. Trends Cell Biol 19, 43-51.
Cosme, J., Liu, P.P., Gramolini, A.O., 2013. The cardiovascular exosome: current perspectives and potential. Proteomics 13, 1654-1659.
Cox, C.S., Jr., Baumgartner, J.E., Harting, M.T., Worth, L.L., Walker, P.A., Shah, S.K., Ewing-Cobbs, L., Hasan, K.M., Day, M.C., Lee, D., Jimenez, F., Gee, A., 2011. Autologous bone marrow mononuclear cell therapy for severe traumatic brain injury in children. Neurosurgery 68, 588-600.
Cvjetkovic, A., Lotvall, J., Lasser, C., 2014. The influence of rotor type and centrifugation time on the yield and purity of extracellular vesicles. J Extracell Vesicles 3.
Denzer, K., Kleijmeer, M.J., Heijnen, H.F., Stoorvogel, W., Geuze, H.J., 2000. Exosome: from internal vesicle of the multivesicular body to intercellular signaling device. J Cell Sci 113 Pt 19, 3365-3374.
Digirolamo, C.M., Stokes, D., Colter, D., Phinney, D.G., Class, R., Prockop, D.J., 1999. Propagation and senescence of human marrow stromal cells in culture: a simple colony-forming assay identifies samples with the greatest potential to propagate and differentiate. Br J Haematol 107, 275-281.
Ding, G.L., Chopp, M., Poulsen, D.J., Li, L., Qu, C., Li, Q., Nejad-Davarani, S.P., Budaj, J.S., Wu, H., Mahmood, A., Jiang, Q., 2013. MRI of neuronal recovery after low-dose methamphetamine treatment of traumatic brain injury in rats. PLoS One 8, e61241.
Dixon, C.E., Clifton, G.L., Lighthall, J.W., Yaghmai, A.A., Hayes, R.L., 1991. A controlled cortical impact model of traumatic brain injury in the rat. J Neurosci Methods 39, 253-262.
Doeppner, T.R., Hermann, D.M., 2014. Stem cell-based treatments against stroke: observations from human proof-of-concept studies and considerations regarding clinical applicability. Front Cell Neurosci 8, 357.
Dominici M, Le Blanc K, Mueller I, Slaper-Cortenbach I, Marini F, Krause D, Deans R, Keating A, Prockop DJ, Horwitz E. Minimal criteria for defining multipotent mesenchymal stromal cells. The International Society for Cellular Therapy position statement. Cytotherapy. 2006;371:1612-1623
Ekanger, L.A., Ali, M.M., Allen, M.J., 2014. Oxidation-responsive Eu(2+/3+)-liposomal contrast agent for dual-mode magnetic resonance imaging. Chem Commun (Camb) 50, 14835-14838.
Ekdahl, C.T., Kokaia, Z., Lindvall, O., 2009. Brain inflammation and adult neurogenesis: the dual role of microglia. Neuroscience 158, 1021-1029.
El-Andaloussi, S., Lee, Y., Lakhal-Littleton, S., Li, J., Seow, Y., Gardiner, C., Alvarez-Erviti, L., Sargent, I.L., Wood, M.J., 2012. Exosome-mediated delivery of siRNA in vitro and in vivo. Nat Protoc 7, 2112-2126.
Gruenberg, J., Griffiths, G., Howell, K.E., 1989. Characterization of the early endosome and putative endocytic carrier vesicles in vivo and with an assay of vesicle fusion in vitro. J Cell Biol 108, 1301-1316.
Gyorgy, B., Szabo, T.G., Pasztoi, M., Pal, Z., Misjak, P., Aradi, B., Laszlo, V., Pallinger, E., Pap, E., Kittel, A., Nagy, G., Falus, A., Buzas, E.I., 2011. Membrane vesicles, current state-of-the-art: emerging role of extracellular vesicles. Cell Mol Life Sci 68, 2667-2688.
Hailer, N.P., 2008. Immunosuppression after traumatic or ischemic CNS damage: it is neuroprotective and illuminates the role of microglial cells. Prog Neurobiol 84, 211-233.
Ho, A.D., Wagner, W., Franke, W., 2008. Heterogeneity of mesenchymal stromal cell preparations. Cytotherapy 10, 320-330.
Imai, T., Takahashi, Y., Nishikawa, M., Kato, K., Morishita, M., Yamashita, T., Matsumoto, A., Charoenviriyakul, C., Takakura, Y., 2015. Macrophage-dependent clearance of systemically administered B16BL6-derived exosomes from the blood circulation in mice. J Extracell Vesicles 4, 26238.
Javazon EH, Beggs KJ, Flake AW. Mesenchymal stem cells: paradoxes of passaging. Exp. Hematol. 2004;32:414-425.
Jin, K., Sun, Y., Xie, L., Peel, A., Mao, X.O., Batteur, S., Greenberg, D.A., 2003. Directed migration of neuronal precursors into the ischemic cerebral cortex and striatum. Mol Cell Neurosci 24, 171-189.
Joyce, N., Annett, G., Wirthlin, L., Olson, S., Bauer, G., Nolta, J.A., 2010. Mesenchymal stem cells for the treatment of neurodegenerative disease. Regen Med 5, 933-946.
Kalani, A., Tyagi, A., Tyagi, N., 2014. Exosomes: mediators of neurodegeneration, neuroprotection and therapeutics. Mol Neurobiol 49, 590-600.
Katsuda, T., Kosaka, N., Takeshita, F., Ochiya, T., 2013. The therapeutic potential of mesenchymal stem cell-derived extracellular vesicles. Proteomics 13, 1637-1653.
Kernie, S.G., Parent, J.M., 2010. Forebrain neurogenesis after focal Ischemic and traumatic brain injury. Neurobiol Dis 37, 267-274.
Kim, H.J., Lee, J.H, Kim, S.H., 2010. Therapeutic effects of human mesenchymal stem cells on traumatic brain injury in rats: secretion of neurotrophic factors and inhibition of apoptosis. J Neurotrauma 27, 131-138.
Kleindienst, A., McGinn, M.J., Harvey, H.B., Colello, R.J., Hamm, R.J., Bullock, M.R., 2005. Enhanced hippocampal neurogenesis by intraventricular S100B infusion is associated with improved cognitive recovery after traumatic brain injury. J Neurotrauma 22, 645-655.
Lai, R.C., Chen, T.S., Lim, S.K., 2011. Mesenchymal stem cell exosome: a novel stem cell-based therapy for cardiovascular disease. Regen Med 6, 481-492.
Lai, R.C., Yeo, R.W., Lim, S.K., 2015. Mesenchymal stem cell exosomes. Semin Cell Dev Biol 40, 82-88.
Lai, R.C., Yeo, R.W., Tan, K.H., Lim, S.K., 2013. Mesenchymal stem cell exosome ameliorates reperfusion injury through proteomic complementation. Regen Med 8, 197-209.
Lakshmipathy, U., Hart, R.P., 2008. Concise review: MicroRNA expression in multipotent mesenchymal stromal cells. Stem Cells 26, 356-363.
Lavoie, J.R., Rosu-Myles, M., 2013. Uncovering the secretes of mesenchymal stem cells. Biochimie 95, 2212-2221.
Li, B., Mahmood, A., Lu, D., Wu, H., Xiong, Y., Qu, C., Chopp, M., 2009. Simvastatin attenuates microglial cells and astrocyte activation and decreases interleukin-lbeta level after traumatic brain injury. Neurosurgery 65, 179-185; discussion 185-176.
Li, L., Chopp, M., Ding, G.L., Qu, C.S., Li, Q.J., Lu, M., Wang, S., Nejad-Davarani, S.P., Mahmood, A., Jiang, Q., 2012. MRI measurement of angiogenesis and the therapeutic effect of acute marrow stromal cell administration on traumatic brain injury. J Cereb Blood Flow Metab 32, 2023-2032.
Li, L., Jiang, Q., Zhang, L., Ding, G., Gang Zhang, Z., Li, Q., Ewing, J.R., Lu, M., Panda, S., Ledbetter, K.A., Whitton, P.A., Chopp, M., 2007. Angiogenesis and improved cerebral blood flow in the ischemic boundary area detected by MRI after administration of sildenafil to rats with embolic stroke. Brain Res 1132, 185-192.
Li, Y., Chen, J., Chen, X.G., Wang, L., Gautam, S.C., Xu, Y.X., Katakowski, M., Zhang, L.J., Lu, M., Janakiraman, N., Chopp, M., 2002. Human marrow stromal cell therapy for stroke in rat: neurotrophins and functional recovery. Neurology 59, 514-523.
Li, Y., Chen, J., Wang, L., Lu, M., Chopp, M., 2001. Treatment of stroke in rat with intracarotid administration of marrow stromal cells. Neurology 56, 1666-1672.
Li, Y., Chopp, M., 2009. Marrow stromal cell transplantation in stroke and traumatic brain injury. Neurosci Lett 456, 120-123.
Liang, X., Ding, Y., Zhang, Y., Tse, H.F., Lian, Q., 2013. Paracrine mechanisms of Mesenchymal Stem cell-based therapy: Current status and perspectives. Cell Transplant.
Liang, X., Ding, Y., Zhang, Y., Tse, H.F., Lian, Q., 2014. Paracrine mechanisms of mesenchymal stem cell-based therapy: current status and perspectives. Cell Transplant 23, 1045-1059.
Lin, B., Ginsberg, M.D., Zhao, W., Alonso, O.F., Belayev, L., Busto, R., 2001. Quantitative analysis of microvascular alterations in traumatic brain injury by endothelial barrier antigen immunohistochemistry. J Neurotrauma 18, 389-397.
Lo, E.H., 2008. A new penumbra: transitioning from injury into repair after stroke. Nat Med 14, 497-500.
Loane, D.J., Stoica, B.A., Faden, A.I., 2015. Neuroprotection for traumatic brain injury. Handb Clin Neurol 127, 343-366.
Lotvall, J., Hill, A.F., Hochberg, F., Buzas, E.I., Di Vizio, D., Gardiner, C., Gho, Y.S., Kurochkin, I.V., Mathivanan, S., Quesenberry, P., Sahoo, S., Tahara, H., Wauben, M.H., Witwer, K.W., Thery, C., 2014. Minimal experimental requirements for definition of extracellular vesicles and their functions: a position statement from the International Society for Extracellular Vesicles. J Extracell Vesicles 3, 26913.
Lu, D., Li, Y., Wang, L., Chen, J., Mahmood, A., Chopp, M., 2001a. Intraarterial administration of marrow stromal cells in a rat model of traumatic brain injury. J Neurotrauma 18, 813-819.
Lu, D., Mahmood, A., Goussev, A., Schallert, T., Qu, C., Zhang, Z.G., Li, Y., Lu, M., Chopp, M., 2004. Atorvastatin reduction of intravascular thrombosis, increase in cerebral microvascular patency and integrity, and enhancement of spatial learning in rats subjected to traumatic brain injury. J Neurosurg 101, 813-821.
Lu, D., Mahmood, A., Qu, C., Goussev, A., Schallert, T., Chopp, M., 2005. Erythropoietin enhances neurogenesis and restores spatial memory in rats after traumatic brain injury. J Neurotrauma 22, 1011-1017.
Lu, D., Mahmood, A., Qu, C., Hong, X., Kaplan, D., Chopp, M., 2007. Collagen scaffolds populated with human marrow stromal cells reduce lesion volume and improve functional outcome after traumatic brain injury. Neurosurgery 61, 596-602; discussion 602-593.
Lu, D., Mahmood, A., Wang, L., Li, Y., Lu, M., Chopp, M., 2001c. Adult bone marrow stromal cells administered intravenously to rats after traumatic brain injury migrate into brain and improve neurological outcome. Neuroreport 12, 559-563.
Mahmood, A., Goussev, A., Lu, D., Qu, C., Xiong, Y., Kazmi, H., Chopp, M., 2008. Long-lasting benefits after treatment of traumatic brain injury (TBI) in rats with combination therapy of marrow stromal cells (MSCs) and simvastatin. J Neurotrauma 25, 1441-1447.
Mahmood, A., Lu, D., Chopp, M., 2004a. Intravenous administration of marrow stromal cells (MSCs) increases the expression of growth factors in rat brain after traumatic brain injury. J Neurotrauma 21, 33-39.
Mahmood, A., Lu, D., Chopp, M., 2004f. Marrow stromal cell transplantation after traumatic brain injury promotes cellular proliferation within the brain. Neurosurgery 55, 1185-1193.
Mahmood, A., Lu, D., Qu, C., Goussev, A., Chopp, M., 2005. Human marrow stromal cell treatment provides long-lasting benefit after traumatic brain injury in rats. Neurosurgery 57, 1026-1031; discussion 1026-1031.
Mahmood, A., Lu, D., Qu, C., Goussev, A., Chopp, M., 2007. Treatment of traumatic brain injury with a combination therapy of marrow stromal cells and atorvastatin in rats. Neurosurgery 60, 546-553; discussion 553-544.
Mahmood, A., Lu, D., Wang, L., Chopp, M., 2002. Intracerebral transplantation of marrow stromal cells cultured with neurotrophic factors promotes functional recovery in adult rats subjected to traumatic brain injury. J Neurotrauma 19, 1609-1617.
Mahmood, A., Lu, D., Wang, L., Li, Y., Lu, M., Chopp, M., 2001. Treatment of traumatic brain injury in female rats with intravenous administration of bone marrow stromal cells. Neurosurgery 49, 1196-1203; discussion 1203-1194.
Mahmood, A., Qu, C., Ning, R., Wu, H., Goussev, A., Xiong, Y., Irtenkauf, S., Li, Y., Chopp, M., 2011. Treatment of TBI with collagen scaffolds and human marrow stromal cells increases the expression of tissue plasminogen activator. J Neurotrauma 28, 1199-1207.
Mahmood, A., Wu, H., Qu, C., Mahmood, S., Xiong, Y., Kaplan, D., Chopp, M., 2014a. Down-regulation of Nogo-A by collagen scaffolds impregnated with bone marrow stromal cell treatment after traumatic brain injury promotes axonal regeneration in rats. Brain Res 1542, 41-48.
Mahmood, A., Wu, H., Qu, C., Mahmood, S., Xiong, Y., Kaplan, D.L., Chopp, M., 2014d. Suppression of neurocan and enhancement of axonal density in rats after treatment of traumatic brain injury with scaffolds impregnated with bone marrow stromal cells. J Neurosurg 120, 1147-1155.
Marklund, N., Hillered, L., 2011. Animal modelling of traumatic brain injury in preclinical drug development: where do we go from here? Br J Pharmacol 164, 1207-1229.
Masyuk, A.I., Masyuk, T.V., Larusso, N.F., 2013. Exosomes in the pathogenesis, diagnostics and therapeutics of liver diseases. J Hepatol 59, 621-625.
McConeghy, K.W., Hatton, J., Hughes, L., Cook, A.M., 2012. A review of neuroprotection pharmacology and therapies in patients with acute traumatic brain injury. CNS Drugs 26, 613-636.
Meng, Y., Xiong, Y., Mahmood, A., Zhang, Y., Qu, C., Chopp, M., 2011. Dose-dependent neurorestorative effects of delayed treatment of traumatic brain injury with recombinant human erythropoietin in rats. J Neurosurg 115, 550-560.
Morgan, R., Kreipke, C.W., Roberts, G., Bagchi, M., Rafols, J.A., 2007. Neovascularization following traumatic brain injury: possible evidence for both angiogenesis and vasculogenesis. Neurol Res 29, 375-381.
Morishita, M., Takahashi, Y., Nishikawa, M., Sano, K., Kato, K., Yamashita, T., Imai, T., Saji, H., Takakura, Y., 2015. Quantitative analysis of tissue distribution of the B16BL6-derived exosomes using a streptavidin-lactadherin fusion protein and iodine-125-labeled biotin derivative after intravenous injection in mice. J Pharm Sci 104, 705-713.
Morris, R.G., Garrud, P., Rawlins, J.N., O'Keefe, J., 1982. Place navigation impaired in rats with hippocampal lesions. Nature 297, 681-683.
Muralidharan-Chari, V., Clancy, J.W., Sedgwick, A., D'Souza-Schorey, C., 2010. Microvesicles: mediators of extracellular communication during cancer progression. J Cell Sci 123, 1603-1611.
Narayan, R.K., Michel, M.E., Ansell, B., Baethmann, A., Biegon, A., Bracken, M.B., Bullock, M.R., Choi, S.C., Clifton, G.L., Contant, C.F., Coplin, W.M., Dietrich, W.D., Ghajar, J., Grady, S.M., Grossman, R.G., Hall, E.D., Heetderks, W., Hovda, D.A., Jallo, J., Katz, R.L., Knoller, N., Kochanek, P.M., Maas, A.I., Majde, J., Marion, D.W., Marmarou, A., Marshall, L.F., McIntosh, T.K., Miller, E., Mohberg, N., Muizelaar, J.P., Pitts, L.H., Quinn, P., Riesenfeld, G., Robertson, C.S., Strauss, K.I., Teasdale, G., Temkin, N., Tuma, R., Wade, C., Walker, M.D., Weinrich, M., Whyte, J., Wilberger, J., Young, A.B., Yurkewicz, L., 2002. Clinical trials in head injury. J Neurotrauma 19, 503-557.
Nichols, J.E., Niles, J.A., Dewitt, D., Prough, D., Parsley, M., Vega, S., Cantu, A., Lee, E., Cortiella, J., 2013. Neurogenic and neuro-protective potential of a novel subpopulation of peripheral blood-derived CD133+ ABCG2+CXCR4+ mesenchymal stem cells: development of autologous cell-based therapeutics for traumatic brain injury. Stem Cell Res Ther 4, 3.
Ning, R., Xiong, Y., Mahmood, A., Zhang, Y., Meng, Y., Qu, C., Chopp, M., 2011. Erythropoietin promotes neurovascular remodeling and long-term functional recovery in rats following traumatic brain injury. Brain Res 1384, 140-150.
Nishibe, M., Barbay, S., Guggenmos, D., Nudo, R.J., 2010. Reorganization of motor cortex after controlled cortical impact in rats and implications for functional recovery. J Neurotrauma 27, 2221-2232.
Ohab, J.J., Fleming, S., Blesch, A., Carmichael, S.T., 2006. A neurovascular niche for neurogenesis after stroke. J Neurosci 26, 13007-13016.
Pant, S., Hilton, H., Burczynski, M.E., 2012. The multifaceted exosome: biogenesis, role in normal and aberrant cellular function, and frontiers for pharmacological and biomarker opportunities. Biochem Pharmacol 83, 1484-1494.
Parent, J.M., Vexler, Z.S., Gong, C., Derugin, N., Ferriero, D.M., 2002. Rat forebrain neurogenesis and striatal neuron replacement after focal stroke. Ann Neurol 52, 802-813.
Paterniti, I., Cordaro, M., Navarra, M., Esposito, E., Cuzzocrea, S., 2015. Emerging pharmacotherapy for treatment of traumatic brain injury: targeting hypopituitarism and inflammation. Expert Opin Emerg Drugs, 1-14.
Paxinos, G., Watson, C., 1986. The rat brain in stereotaxic coordinates, 2nd ed. Academic Press, Sydney ; Orlando.
Peng, W., Sun, J., Sheng, C., Wang, Z., Wang, Y., Zhang, C., Fan, R., 2015. Systematic review and meta-analysis of efficacy of mesenchymal stem cells on locomotor recovery in animal models of traumatic brain injury. Stem Cell Res Ther 6, 47.
Phinney DG, Prockop DJ. Concise review: mesenchymal stem/multipotent stromal cells: the state of transdifferentiation and modes of tissue repair--current views. Stem Cells. 2007; 25:2896-2902.
Qu, C., Mahmood, A., Liu, X.S., Xiong, Y., Wang, L., Wu, H., Li, B., Zhang, Z.G., Kaplan, D.L., Chopp, M., 2011. The treatment of TBI with human marrow stromal cells impregnated into collagen scaffold: functional outcome and gene expression profile. Brain Res 1371, 129-139.
Qu, C., Mahmood, A., Lu, D., Goussev, A., Xiong, Y., Chopp, M., 2008. Treatment of traumatic brain injury in mice with marrow stromal cells. Brain Res 1208, 234-239.
Qu, C., Xiong, Y., Mahmood, A., Kaplan, D.L., Goussev, A., Ning, R., Chopp, M., 2009. Treatment of traumatic brain injury in mice with bone marrow stromal cell-impregnated collagen scaffolds. J Neurosurg 111, 658-665.
Richardson, R.M., Sun, D., Bullock, M.R., 2007. Neurogenesis after traumatic brain injury. Neurosurg Clin N Am 18, 169-181.
Sanchez-Ramos J, Song S, Cardozo-Pelaez F, Hazzi C, Stedeford T, Willing A, Freeman TB, Saporta S, Janssen W, Patel N, Cooper DR, Sanberg PR. Adult bone marrow stromal cells differentiate into neural cells in vitro. Exp Neurol. 2000; 164:247-256.
Schwab, J.M., Beschorner, R., Nguyen, T.D., Meyermann, R., Schluesener, H.J., 2001. Differential cellular accumulation of connective tissue growth factor defines a subset of reactive astrocytes, invading fibroblasts, and endothelial cells following central nervous system injury in rats and humans. J Neurotrauma 18, 377-388.
Simons, M., Raposo, G., 2009. Exosomes--vesicular carriers for intercellular communication. Curr Opin Cell Biol 21, 575-581.
Singh, S., Swarnkar, S., Goswami, P., Nath, C., 2011. Astrocytes and microglia: responses to neuropathological conditions. Int J Neurosci 121, 589-597.
Skolnick, B.E., Maas, A.I., Narayan, R.K., van der Hoop, R.G., MacAllister, T., Ward, J.D., Nelson, N.R., Stocchetti, N., Investigators, S.T., 2014. A clinical trial of progesterone for severe traumatic brain injury. N Engl J Med 371, 2467-2476.
Smith, J.M., Lunga, P., Story, D., Harris, N., Le Belle, J., James, M.F., Pickard, J.D., Fawcett, J.W., 2007. Inosine promotes recovery of skilled motor function in a model of focal brain injury. Brain 130, 915-925.
Stoorvogel, W., Kleijmeer, M.J., Geuze, H.J., Raposo, G., 2002. The biogenesis and functions of exosomes. Traffic 3, 321-330.
Sun, D., McGinn, M.J., Zhou, Z., Harvey, H.B., Bullock, M.R., Colello, R.J., 2007. Anatomical integration of newly generated dentate granule neurons following traumatic brain injury in adult rats and its association to cognitive recovery. Exp Neurol 204, 264-272.
Sundholm-Peters, N.L., Yang, H.K., Goings, G.E., Walker, A.S., Szele, F.G., 2005. Subventricular zone neuroblasts emigrate toward cortical lesions. J Neuropathol Exp Neurol 64, 1089-1100.
Swanson, R.A., Morton, M.T., Tsao-Wu, G., Savalos, R.A., Davidson, C., Sharp, F.R., 1990. A semiautomated method for measuring brain infarct volume. J Cereb Blood Flow Metab 10, 290-293.
Takahashi, Y., Nishikawa, M., Shinotsuka, H., Matsui, Y., Ohara, S., Imai, T., Takakura, Y., 2013. Visualization and in vivo tracking of the exosomes of murine melanoma B16-BL6 cells in mice after intravenous injection. J Biotechnol 165, 77-84.
Taupin, P., 2006. The therapeutic potential of adult neural stem cells. Curr Opin Mol Ther 8, 225-231.
Tauro, B.J., Greening, D.W., Mathias, R.A., Ji, H., Mathivanan, S., Scott, A.M., Simpson, R.J., 2012. Comparison of ultracentrifugation, density gradient separation, and immunoaffinity capture methods for isolating human colon cancer cell line LIM1863-derived exosomes. Methods 56, 293-304.
Taylor, D.D., Zacharias, W., Gercel-Taylor, C., 2011. Exosome isolation for proteomic analyses and RNA profiling. Methods Mol Biol 728, 235-246.
Thai, S.C., Neuhaus, W., 2014. The blood-brain barrier as a target in traumatic brain injury treatment. Arch Med Res 45, 698-710.
Tsai, M.J., Tsai, S.K., Hu, B.R., Liou, D.Y., Huang, S.L., Huang, M.C., Huang, W.C., Cheng, H., Huang, S.S., 2014. Recovery of neurological function of ischemic stroke by application of conditioned medium of bone marrow mesenchymal stem cells derived from normal and cerebral ischemia rats. J. Biomed. Sci. 21, 5.
Uccelli A, Moretta L, Pistoia V. Mesenchymal stem cells in health and disease. Nat. Rev. Immunol. 2008; 8:726-736.
van Doormaal, F.F., Kleinjan, A., Di Nisio, M., Buller, H.R., Nieuwland, R., 2009. Cell-derived microvesicles and cancer. Neth J Med 67, 266-273.
Villarroya-Beltri, C., Gutierrez-Vazquez, C., Sanchez-Cabo, F., Perez-Hernandez, D., Vazquez, J., Martin-Cofreces, N., Martinez-Herrera, D.J., Pascual-Montano, A., Mittelbrunn, M., Sanchez-Madrid, F., 2013. Sumoylated hnRNPA2B1 controls the sorting of miRNAs into exosomes through binding to specific motifs. Nat Commun 4, 2980.
Vink, R., Nimmo, A.J., 2009. Multifunctional drugs for head injury. Neurotherapeutics 6, 28-42.
Vlassov, A.V., Magdaleno, S., Setterquist, R., Conrad, R., 2012. Exosomes: current knowledge of their composition, biological functions, and diagnostic and therapeutic potentials. Biochim Biophys Acta 1820, 940-948.
Walker, P.A., Shah, S.K., Harting, M.T., Cox, C.S., Jr., 2009. Progenitor cell therapies for traumatic brain injury: barriers and opportunities in translation. Dis Model Mech 2, 23-38.
Walker, P.A., Shah, S.K., Jimenez, F., Aroom, K.R., Harting, M.T., Cox, C.S., Jr., 2012. Bone marrow-derived stromal cell therapy for traumatic brain injury is neuroprotective via stimulation of non-neurologic organ systems. Surgery 152, 790-793.
Woodcock, T., Morganti-Kossmann, M.C., 2013. The role of markers of inflammation in traumatic brain injury. Front Neurol 4, 18.
Wright, D.W., Yeatts, S.D., Silbergleit, R., Palesch, Y.Y., Hertzberg, V.S., Frankel, M., Goldstein, F.C., Caveney, A.F., Howlett-Smith, H., Bengelink, E.M., Manley, G.T., Merck, L.H., Janis, L.S., Barsan, W.G., Investigators, N., 2014. Very early administration of progesterone for acute traumatic brain injury. N Engl J Med 371, 2457-2466.
Xin, H., Chopp, M., Shen, L.H., Zhang, R.L., Zhang, L., Zhang, Z.G., Li, Y., 2013a. Multipotent mesenchymal stromal cells decrease transforming growth factor beta1 expression in microglia/macrophages and down-regulate plasminogen activator inhibitor 1 expression in astrocytes after stroke. Neurosci Lett 542, 81-86.
Xin, H., Li, Y., Buller, B., Katakowski, M., Zhang, Y., Wang, X., Shang, X., Zhang, Z.G., Chopp, M., 2012. Exosome-mediated transfer of miR-133b from multipotent mesenchymal stromal cells to neural cells contributes to neurite outgrowth. Stem Cells 30, 1556-1564.
Xin, H., Li, Y., Chopp, M., 2014. Exosomes/miRNAs as mediating cell-based therapy of stroke. Front Cell Neurosci 8, 377.
Xin, H., Li, Y., Cui, Y., Yang, J.J., Zhang, Z.G., Chopp, M., 2013b. Systemic administration of exosomes released from mesenchymal stromal cells promote functional recovery and neurovascular plasticity after stroke in rats. J Cereb Blood Flow Metab 33, 1711-1715.
Xin, H., Li, Y., Liu, Z., Wang, X., Shang, X., Cui, Y., Zhang, Z.G., Chopp, M., 2013g. MiR-133b Promotes Neural Plasticity and Functional Recovery After Treatment of Stroke with Multipotent Mesenchymal Stromal Cells in Rats Via Transfer of Exosome-Enriched Extracellular Particles. Stem Cells 31, 2737-2746.
Xiong, Y., Mahmood, A., Chopp, M., 2010. Angiogenesis, neurogenesis and brain recovery of function following injury. Curr Opin Investig Drugs 11, 298-308.
Xiong, Y., Mahmood, A., Chopp, M., 2013. Animal models of traumatic brain injury. Nat Rev Neurosci 14, 128-142.
Xiong, Y., Mahmood, A., Meng, Y., Zhang, Y., Zhang, Z.G., Morris, D.C., Chopp, M., 2011a. Treatment of traumatic brain injury with thymosin beta(4) in rats. J Neurosurg 114, 102-115.
Xiong, Y., Mahmood, A., Zhang, Y., Meng, Y., Zhang, Z.G., Qu, C., Sager, T.N., Chopp, M., 2011b. Effects of posttraumatic carbamylated erythropoietin therapy on reducing lesion volume and hippocampal cell loss, enhancing angiogenesis and neurogenesis, and improving functional outcome in rats following traumatic brain injury. J Neurosurg 114, 549-559.
Xiong, Y., Qu, C., Mahmood, A., Liu, Z., Ning, R., Li, Y., Kaplan, D.L., Schallert, T., Chopp, M., 2009. Delayed transplantation of human marrow stromal cell-seeded scaffolds increases transcallosal neural fiber length, angiogenesis, and hippocampal neuronal survival and improves functional outcome after traumatic brain injury in rats. Brain Res 1263, 183-191.
Xiong, Y., Zhang, Y., Mahmood, A., Meng, Y., Qu, C., Chopp, M., 2011c. Erythropoietin mediates neurobehavioral recovery and neurovascular remodeling following traumatic brain injury in rats by increasing expression of vascular endothelial growth factor. Transl Stroke Res 2, 619-632.
Xu, L., Yang, B.F., Ai, J., 2013. MicroRNA transport: a new way in cell communication. J Cell Physiol 228, 1713-1719.
Yang, T., Martin, P., Fogarty, B., Brown, A., Schurman, K., Phipps, R., Yin, V.P., Lockman, P., Bai, S., 2015. Exosome delivered anticancer drugs across the blood-brain barrier for brain cancer therapy in Danio rerio. Pharm Res 32, 2003-2014.
Yu, B., Zhang, X., Li, X., 2014. Exosomes derived from mesenchymal stem cells. Int J Mol Sci 15, 4142-4157.
Zhang, R., Liu, Y., Yan, K., Chen, L., Chen, X.R, Li, P., Chen, F.F., Jiang, X.D., 2013a. Anti-inflammatory and immunomodulatory mechanisms of mesenchymal stem cell transplantation in experimental traumatic brain injury. J Neuroinflammation 10, 106.
Zhang, Y., Chopp, M., Mahmood, A., Meng, Y., Qu, C., Xiong, Y., 2012. Impact of inhibition of erythropoietin treatment-mediated neurogenesis in the dentate gyrus of the hippocampus on restoration of spatial learning after traumatic brain injury. Exp Neurol 235, 336-344.
Zhang, Y., Chopp, M., Meng, Y., Katakowski, M., Xin, H., Mahmood, A., Xiong, Y., 2015. Effect of exosomes derived from multipluripotent mesenchymal stromal cells on functional recovery and neurovascular plasticity in rats after traumatic brain injury. J Neurosurg 122, 856-867.
Zhang, Y., Chopp, M., Meng, Y., Zhang, Z.G., Doppler, E., Mahmood, A., Xiong, Y., 2013b. Improvement in functional recovery with administration of Cerebrolysin after experimental closed head injury. J Neurosurg 118, 1343-1355.
Zhang, Z.G., Chopp, M., 2015. Promoting brain remodeling to aid in stroke recovery. Trends Mol Med 21, 543-548.
Zhang, Z.X., Guan, L.X., Zhang, K., Zhang, Q., Dai, L.J., 2008. A combined procedure to deliver autologous mesenchymal stromal cells to patients with traumatic brain injury. Cytotherapy 10, 134-139.
Zheng, W., ZhuGe, Q., Zhong, M., Chen, G., Shao, B., Wang, H., Mao, X., Xie, L., Jin, K., 2013. Neurogenesis in adult human brain after traumatic brain injury. J Neurotrauma 30, 1872-1880.
Where the claims recite "a" or "a first" element of the equivalent thereof, such claims should be understood to include incorporation of one or more such elements, neither requiring nor excluding two or more such elements.

## Claims

1. A composition comprising exosomes obtained from human mesenchymal stem cells grown in three-dimensional cell culture comprising three-dimensional collagen scaffolds for use in treating a subject suffering from a traumatic brain injury (TBI).

2. A composition for use, according to claim 1, wherein the composition comprises from about 1 x 10⁹ to about 1 x 10¹⁵ exosomes per kg body weight of the subject.

3. A composition for use, according to claim 1 or claim 2, wherein the composition is administered to the subject intravenously, subcutaneously, cutaneously, intraperitoneally, intraarterially, intracerebrally, intrathecally, intracerebroventricularly, or intranasally.

4. A composition for use, according to claim 1, wherein the composition is administered within 24 hours of developing the TBI.

5. A composition for use, according to claim 1, wherein treating a subject suffering from a traumatic brain injury comprises treating or preventing one or more symptoms of TBI.

6. A composition for use, according to claim 5, wherein one or more symptoms of TBI include one or more of:
(i) any period of loss of or a decreased level of consciousness;
(ii) loss of memory for events immediately before or after the injury;
(iii) a neurologic deficit, selected from the group consisting of: weakness, loss of balance, change in vision, dyspraxia, paresis, plegia, sensory loss, and aphasia;
(iv) an alteration in mental state at the time of the injury selected from confusion, disorientation, and slowed thinking;
(v) visual, neuroradiologic, or laboratory confirmation of damage to the brain;
(vi) Difficulty with long-term or short-term memory;
(vii) Difficulty in maintaining attention and concentration;
(viii) Difficulty with flexibility in thinking, reasoning and problem-solving;
(ix) Difficulty with orientation to person, places and/or time;
(x) Difficulty with speed of processing information;
(xi) Difficulty in initiating, maintaining, restructuring and terminating conversation; Difficulty in maintaining the topic of conversation; Difficulty in discriminating relevant from irrelevant information; Difficulty in producing relevant speech; Difficulty responding to verbal communication in a timely, accurate, and efficient manner; Difficulty in understanding verbal information;
(xii) Difficulty with word retrieval; Difficulty with articulation; Difficulty with voice production selected from the group consisting of intensity, pitch and/or quality of voice; Difficulty in producing fluent speech; Difficulty in formulating and sequencing ideas; Difficulty with abstract and figurative language; Difficulty with perseverated speech; Difficulty using appropriate syntax;
(xiii) Difficulty in understanding and producing written communication;
(xiv) Difficulty with noise overload;
(xv) Difficulty in interpreting subtle verbal and nonverbal cues during conversation;
(xvi) Difficulty in initiating and sustaining appropriate peer and family relationships;
(xvii) Difficulty in coping with over-stimulating environments;
(xviii) Difficulty with using self-control selected from verbal and physical aggression;
(xix) Difficulty with speaking and acting impulsively;
(xx) Difficulty in initiating activities; Difficulty in adjusting to change; Difficulty in compliance with requests;
(xxi) Hyperactivity; Intensification of pre-existent maladaptive behaviors and/or disabilities; Short-term or long-term physical disabilities; seizure activity; Difficulty in spatial orientation; Difficulty with mobility and independence; Problems in balance, strength, muscle tone, equilibrium and gross motor skills; Vertigo; Hearing loss and/or auditory processing problems; Difficulty with fine motor skills; Difficulty in speed of processing and motor response time; Difficulty with bowel and/or bladder control; premature puberty; Loss of stamina and/or sense of fatigue; and Difficulty in administering self-care.

7. A composition for use, according to claim 1, wherein treating a subject suffering from a TBI comprises at least one or more of: (1) promotion of sensorimotor functional recovery; (2) enhancement of spatial learning; (3) an increase in vascular density and angiogenesis; (4) increased neurogenesis in the DG; and (5) significant reduction in brain inflammation.

## Patentansprüche

1. Zusammensetzung, die Exosome umfasst, die aus humanen mesenchymalen Stammzellen erhalten werden, die in einer dreidimensionalen Zellkultur gezüchtet werden, die dreidimensionale Kollagengerüste umfasst, zur Verwendung bei der Behandlung eines Subjekts, das an einer traumatischen Hirnverletzung (traumatic brain injury - TBI) leidet.

2. Zusammensetzung zur Verwendung nach Anspruch 1, wobei die Zusammensetzung von etwa 1 x 10⁹ bis etwa 1 x 10¹⁵ Exosome pro kg Körpergewicht des Subjekts umfasst.

3. Zusammensetzung zur Verwendung nach Anspruch 1 oder 2, wobei die Zusammensetzung dem Subjekt intravenös, subkutan, kutan, intraperitoneal, intraarteriell, intrazerebral, intrathekal, intrazerebroventrikulär oder intranasal verabreicht wird.

4. Zusammensetzung zur Verwendung nach Anspruch 1, wobei die Zusammensetzung innerhalb von 24 Stunden nach Entwickeln der TBI verabreicht wird.

5. Zusammensetzung zur Verwendung nach Anspruch 1, wobei ein Behandeln eines Subjekts, das an einer traumatischen Hirnverletzung leidet, das Behandeln oder ein Vorbeugen von einem oder mehreren Symptomen von TBI umfasst.

6. Zusammensetzung zur Verwendung nach Anspruch 5, wobei ein oder mehrere Symptome von TBI Folgendes beinhaltet:
(i) eine beliebige Periode von Verlust von oder einem verminderten Bewusstsein;
(ii) Gedächtnisverlust für Ereignisse unmittelbar vor oder nach der Verletzung;
(iii) ein neurologisches Defizit, ausgewählt aus der Gruppe, die aus Folgendem besteht: Schwäche, Gleichgewichtsverlust, Veränderung der Sehkraft, Dyspraxie, Parese, Plegie, sensorischer Verlust und Aphasie;
(iv) einen Wechsel des Geisteszustands zu dem Zeitpunkt der Verletzung, ausgewählt aus Verwirrtheit, Desorientiertheit und verlangsamtem Denken;
(v) visuelle, neuroradiologische oder Laborbestätigung einer Schädigung des Hirns;
(vi) Schwierigkeiten mit dem Langzeit- oder Kurzzeitgedächtnis;
(vii) Schwierigkeiten bei einem Aufrechterhalten von Aufmerksamkeit und Konzentration;
(viii) Schwierigkeiten mit einer Flexibilität des Denkens, Argumentieren und Problemlösen;
(ix) Schwierigkeiten mit einer Orientierung zu Personen, Orten und/oder Zeit;
(x) Schwierigkeiten mit der Geschwindigkeit bei dem Verarbeiten von Informationen;
(xi) Schwierigkeiten bei dem Initiieren, Aufrechterhalten, Umstrukturieren und Beenden von Gesprächen; Schwierigkeiten bei dem Aufrechterhalten eines Gesprächsthemas; Schwierigkeiten bei dem Unterscheiden von relevanten und irrelevanten Informationen; Schwierigkeiten bei dem Produzieren relevanter Sprache; Schwierigkeiten, auf verbale Kommunikation in rechtzeitiger, genauer und effizienter Weist zu reagieren; Schwierigkeiten bei dem Verstehen verbaler Informationen;
(xii) Schwierigkeiten mit dem Abrufen von Wörtern; Schwierigkeiten mit der Artikulation; Schwierigkeiten mit der Stimmproduktion ausgewählt aus der Gruppe, die aus Intensität, Tonhöhe und/oder Qualität der Stimme besteht; Schwierigkeiten bei dem Produzieren von fließender Sprache; Schwierigkeiten bei dem Formulieren und Sequenzieren von Ideen; Schwierigkeiten mit abstrakter und bildlicher Sprache; Schwierigkeiten mit beharrlicher Sprache; Schwierigkeiten bei dem Anwenden von angemessener Syntax;
(xiii) Schwierigkeiten bei dem Verstehen und Produzieren schriftlicher Kommunikation;
(xiv) Schwierigkeiten mit Lärmüberlastung;
(xv) Schwierigkeiten bei dem Interpretieren subtiler verbaler und nonverbaler Hinweise während eines Gesprächs;
(xvi) Schwierigkeiten bei dem Aufbauen und Erhalten von angemessenen Beziehungen zu Gleichaltrigen und der Familie;
(xvii) Schwierigkeiten bei dem Bewältigen von überreizenden Umgebungen;
(xviii) Schwierigkeiten mit dem Anwenden von Selbstbeherrschung, ausgewählt aus verbaler und körperlicher Aggression;
(xix) Schwierigkeiten mit impulsiven Sprechen und Handeln;
(xx) Schwierigkeiten bei dem Initiieren von Aktivitäten; Schwierigkeiten bei dem Anpassen an Veränderungen; Schwierigkeiten bei einer Erfüllung von Anforderungen; und/oder
(xxi) Hyperaktivität; Intensivierung bereits bestehender maladaptiver Verhaltensweisen und/oder Behinderungen; Kurzfristige oder langfristige körperliche Behinderungen; Krampfanfälle; Schwierigkeiten bei räumlicher Orientierung; Schwierigkeiten mit Mobilität und Unabhängigkeit; Probleme mit Gleichgewicht, Kraft, Muskeltonus, Balance und grobmotorischen Fähigkeiten; Schwindel; Hörverlust und/oder auditive Verarbeitungsprobleme; Schwierigkeiten mit feinmotorischen Fähigkeiten; Schwierigkeiten bei der Verarbeitungsgeschwindigkeit und der motorischen Reaktionszeit; Schwierigkeiten mit Darm- und/oder Blasenkontrolle; vorzeitige Pubertät; Verlust von Ausdauer und/oder Ermüdungsgefühl; und Schwierigkeiten bei einem Ausüben der Selbstversorgung.

7. Zusammensetzung zur Verwendung nach Anspruch 1, wobei das Behandeln eines Subjekts, das an einer TBI leidet, mindestens eins oder mehrere des Folgenden umfasst: (1) Förderung der sensomotorischen funktionellen Erholung; (2) Verbesserung des räumlichen Lernens; (3) eine Erhöhung der Gefäßdichte und Angiogenese; (4) erhöhte Neurogenese in dem DG; und/oder (5) signifikante Verringerung der Hirnentzündung.

## Revendications

1. Composition comprenant des exosomes obtenus à partir de cellules souches mésenchymateuses humaines cultivées dans une culture cellulaire tridimensionnelle comprenant des échafaudages de collagène tridimensionnels, destinée à être utilisée dans le traitement d'un sujet souffrant d'un traumatisme crânio-cérébral (TCC).

2. Composition destinée à être utilisée selon la revendication 1, dans laquelle la composition comprend environ 1 x 10⁹ à environ 1 x 10¹⁵ exosomes par kg de poids corporel du sujet.

3. Composition destinée à être utilisée selon la revendication 1 ou la revendication 2, dans laquelle la composition est administrée au sujet par voie intraveineuse, sous-cutanée, cutanée, intrapéritonéale, intra-artérielle, intracérébrale, intrathécale, intracérébroventriculaire ou intranasale.

4. Composition destinée à être utilisée selon la revendication 1, dans laquelle la composition est administrée dans les 24 heures suivant le développement du TCC.

5. Composition destinée à être utilisée selon la revendication 1, dans laquelle le traitement d'un sujet souffrant d'un traumatisme crânio-cérébral comprend le traitement ou la prévention d'un ou de plusieurs symptômes de TCC.

6. Composition destinée à être utilisée selon la revendication 5, dans laquelle un ou plusieurs symptômes de TCC comportent :
(i) toute période de perte ou de diminution du niveau de conscience ; et/ou
(ii) une perte de mémoire pour des événements immédiatement avant ou après le traumatisme ; et/ou
(iii) un déficit neurologique, choisi dans le groupe constitué par: la faiblesse, la perte d'équilibre, la modification de la vision, la dyspraxie, la parésie, la plégie, la perte sensorielle et l'aphasie ; et/ou
(iv) une altération de l'état mental au moment du traumatisme choisie parmi la confusion, la désorientation et un ralentissement de la pensée ; et/ou
(v) la confirmation visuelle, neuroradiologique ou en laboratoire de lésions cérébrales ; et/ou
(vi) des difficultés avec la mémoire à long terme ou à court terme ; et/ou
(vii) des difficultés à maintenir l'attention et la concentration ; et/ou
(viii) des difficultés à penser, à raisonner et à résoudre des problèmes avec souplesse ; et/ou
(ix) des difficultés à s'orienter vers des personnes, des lieux et/ou dans le temps ; et/ou
(x) des difficultés à traiter des informations rapidement ; et/ou
(xi) des difficultés à initier, à maintenir, à restructurer et à terminer une conversation ; et/ou des difficultés à maintenir le sujet de la conversation ; et/ou des difficultés à distinguer des informations pertinentes d'informations non pertinentes ; et/ou des difficultés à produire un discours pertinent ; et/ou des difficultés à répondre à une communication verbale de manière opportune, précise et efficace ; et/ou des difficultés à comprendre des informations verbales ; et/ou
(xii) des difficultés à récupérer des mots ; et/ou des difficultés à articuler ; et/ou des difficultés à produire des sons choisies dans le groupe constitué par l'intensité, la hauteur et/ou la qualité de la voix; et/ou des difficultés à produire un discours fluide ; et/ou des difficultés à formuler et à séquencer des idées ; et/ou des difficultés avec les langages abstrait et figuratif ; et/ou des difficultés à poursuivre un discours ; et/ou des difficultés à utiliser une syntaxe appropriée ; et/ou
(xiii) des difficultés à comprendre et à produire une communication écrite ; et/ou
(xiv) des difficultés avec la surcharge sonore ; et/ou
(xv) des difficultés à interpréter des signaux verbaux et non verbaux subtils pendant une conversation ; et/ou
(xvi) des difficultés à initier et à entretenir des relations appropriées avec les pairs et la famille ; et/ou
(xvii) des difficultés à faire face à des environnements trop stimulants ; et/ou
(xviii) des difficultés à utiliser la maîtrise de soi choisies parmi les agressions verbales et physiques ; et/ou
(xix) des difficultés à parler et à agir de manière impulsive ; et/ou
(xx) des difficultés à initier des activités ; et/ou des difficultés à s'adapter au changement ; et/ou des difficultés à se conformer à des demandes ; et/ou
(xxi) l'hyperactivité ; et/ou une intensification de comportements inadaptés et/ou d'handicaps préexistants ; et/ou des handicaps physiques de courte ou de longue durée ; et/ou des crises d'épilepsie ; et/ou des difficultés à s'orienter dans l'espace ; et/ou des difficultés avec la mobilité et l'autonomie ; et/ou des problèmes d'équilibre, de force, de tonus musculaire, de stabilité et de motricité globale ; et/ou des vertiges ; et/ou une perte auditive et/ou des problèmes de traitement auditif ; et/ou des difficultés avec la motricité fine ; et/ou des difficultés avec la vitesse de traitement et le temps de réponse motrice ; et/ou des difficultés à contrôler les intestins et/ou la vessie ; et/ou une puberté prématurée ; et/ou une perte d'endurance et/ou une sensation de fatigue ; et/ou des difficultés à administrer les soins personnels.

7. Composition destinée à être utilisée selon la revendication 1, dans laquelle le traitement d'un sujet souffrant d'un TCC comprend un ou plusieurs de : (1) la promotion de la récupération fonctionnelle sensorimotrice ; et/ou (2) l'amélioration de l'apprentissage spatial ; et/ou (3) une augmentation de la densité vasculaire et de l'angiogenèse ; et/ou (4) une augmentation de la neurogenèse dans le DG ; et/ou (5) une réduction significative de l'inflammation cérébrale.
